# EUROPEAN PATENT APPLICATION

(11) **EP 4 169 529 A1**
(43) Date of publication of application: **26.04.2023**
(21) Application number: 21382943.5
(22) Date of filing: 20.10.2021
(51) Int. Cl.: A61K 39/108, A61K 39/104, A61P 31/04

(54) **LIPOPOLYSACCHARIDE (LPS) DEFICIENT ACINETOBACTER BAUMANNII MULTIVALENT VACCINE**

(71) Applicant: Vaxdyn S.L., 41704 Dos Hermanas, Sevilla (ES); Instituto de Salud Carlos III, 28029 Madrid (ES)
(72) Inventor: INFANTE VIÑOLO, Juan José, 41704 Sevilla (ES); McConnell, Michael James, 28220 Madrid (ES); RODRÍGUEZ ROSADO, Ana Isabel, 41704 Sevilla (ES); CORDERO ALBA, María del Mar, 41704 Sevilla (ES); PÉREZ GÓMEZ, Astrid, 41704 Seville (ES); CORRAL LUGO, Andrés, 28220 Madrid (ES)
(74) Representative: Hoffmann Eitle

(57) **Abstract**

**Lipopolysaccharide (LPS) deficient *Acinetobacter* baumannii multivalent vaccine.** The invention refers to a composition comprising inactivated cells deficient in LPS from the genus *Acinetobacter* and/or outer membrane vesicles form the same and their use for the manufacture of a medicament, preferably a vaccine, for the prevention of diseases caused by *K. pneumoniae* and/or *P. aeruginosa* and optionally *A baumannii.*

## Description

### Technical field

The invention refers to a composition comprising inactivated cells deficient in LPS from the genus *Acinetobacter* and/or outer membrane vesicles form the same and their use for the manufacture of a medicament, preferably a vaccine, for the prevention of diseases caused by *Klebsiella pneumoniae* and/or *Pseudomonas aeruginosa* and optionally *Acinetobacter baumannii.*

### Background Art

The management of carbapenem-resistant infections is often based on polymyxins, tigecycline, aminoglycosides and their combinations. However, in a recent reviews, we have found that Gram-negative bacteria (GNB) co-resistant to carbapanems, aminoglycosides, polymyxins and tigecycline (CAPT-resistant) are increasingly being reported worldwide. There is thus a need for further treatment options in combination with rapid diagnostic methods. In this invention, we specifically focus on three of the most problematic species, namely *K. pneumoniae, P. aeruginosa* and *A. baumannii.* Several treatment options are currently available for CAPT-resistant *K*. *pneumonia.* Newer β-lactam-β-lactamase combinations, including the combination of ceftazidime/avibactam with aztreonam against metallo-β-lactamase-producing isolates, appear to be more effective compared to combinations of older agents. Options for P. aeruginosa (especially metallo-β-lactamase-producing strains) and *A. baumannii* remain limited. Synergistic combination of older agents (e.g., polymyxin- or fosfomycin-based synergistic combinations) may represent a last resort option, but their use against CAPT-resistant GNB requires further study. This, again, highlights the urgent need for further therapeutic options.

### Brief description of the drawings

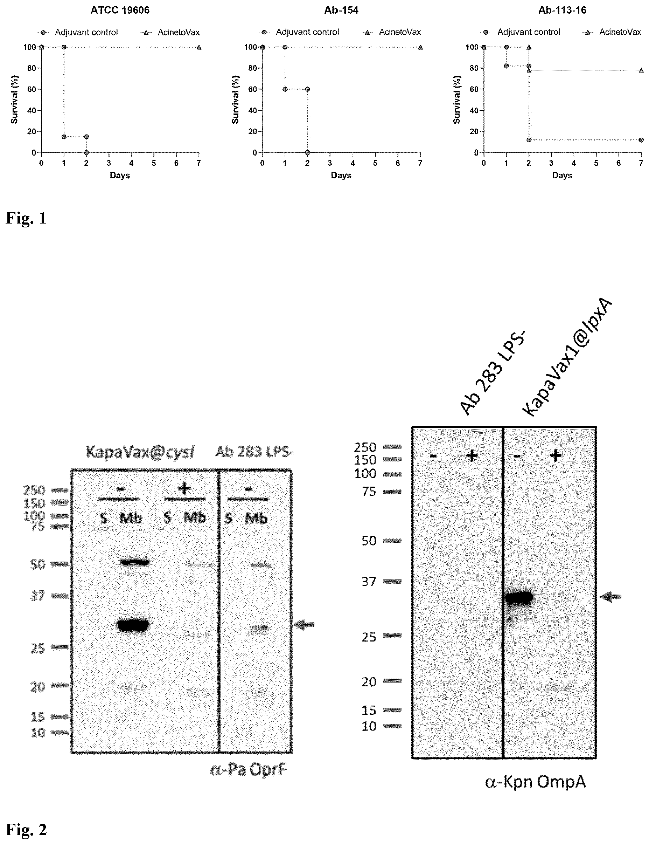

**Fig. 1****.** This figure shows that AcinetoVax, a vaccine based on inactivated whole-cells of an LPS-deficient *A. baumannii* strain characterized by the complete inactivation of *lpxC* as taught in EP2942389A1 plus the adjuvant aluminum hydroxide, protects against infection by A. baumannii clinical isolates. Mice were infected with the indicated strains of *A. baumannii* (ATCC 19606, Ab-154, and Ab-113-16) 7 days after the second immunization with AcinetoVax (day 21) and survival was monitored for 7 days. ATCC 19606 is a urine infection clinical isolate used as reference strain in sepsis and pneumonia models of infection in mice. Ab-154 is a clinical isolate sensitive to carbapenems, from an outbreak at the Hospital Virgen del Rocío of Seville in 2002. Ab-113-16 is a panresistant clinical isolate from a patient who died as a consequence of the 2002 outbreak.
**Fig. 2****.** Surface exposure of heterologous antigens in LPS-deficient *A. baumannii* cells edited for expression of *K. pneumoniae* OmpA and OpmK36 and *P. aeruginosa* OprF and fusion protein OprI::PcrV (KapaVax, left) and in LPS-deficient *A. baumannii* cells edited for expression of *K. pneumoniae* OmpA (KapaVax1, right) . Cultures were washed after growth, resuspended in PBS and treated with 0.5 mg/ml of Proteinase K for 1h at 37°C. Western-Blot with polyclonal anti-sera against recombinant protein Pa-OprF (left) or Kp-OmpA (right). Treatment with Proteinase K is indicated by + (treated) or - (untreated) in each sample). Mb: Outer-Membrane extract; S: extract supernatant. Protocol obtained from Richard N. Besingi and Patricia L. Clark. Nature Protocols. 2015 Dec; 10(12):2074-2080. Sensitivity to treatment shows surface exposure of the proteins.

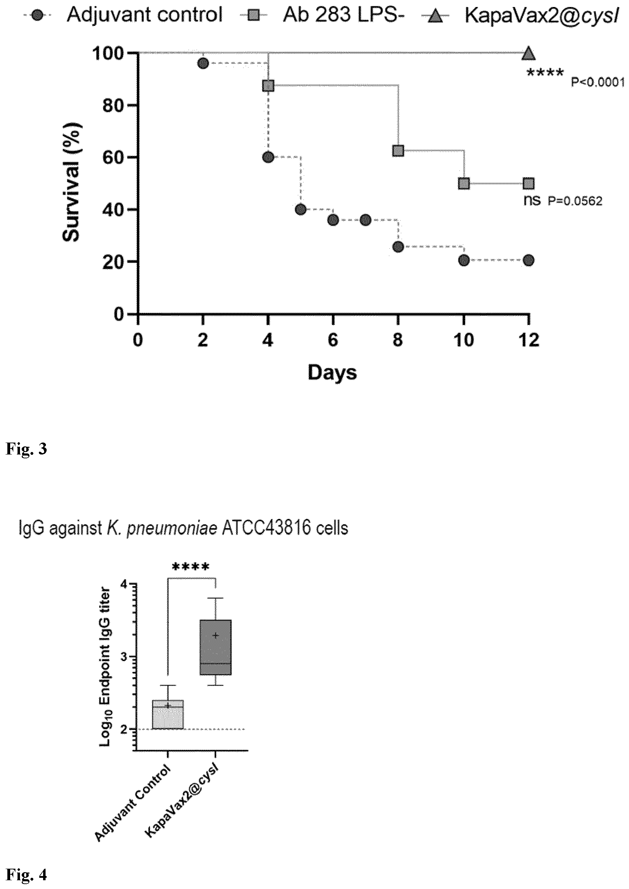

**Fig. 3** shows a survival plot of murine sepsis model by *K. pneumoniae* infection. Animals were immunized with 2 doses in alternate weeks with KapaVax2 (N=18), the carrier Ab283 LPS- cells (N=8) or vehicle alone (N=25). IP challenge with the strain ATCC43816 (day 7 after 2nd immunization). The plot merged data from 3 independent experiments, with challenge doses 8.5x10³, 6.7x10³, and 6.7x10³ cfu, respectively. Survival was monitored for 7-12 days. Statistical differences were measured by Log-rank (Mantel-Cox) test (ns stands for non-significant). *K. pneumoniae* ATCC43816 is a hypervirulent, hypercapsulated clinical isolate, producing acute pneumonia and systemic spread
**Fig 4** shows IgG levels against *K. pneumoniae* cells. ELISA recognition of *K. pneumoniae* ATCC43816 cells by antisera from animals immunized with 2 doses of KapaVax2 (N=10) or vehicle alone (N=10), sampled at day 21. Statistical analysis by 2-tailed Mann-Whitney test (ns, p>0,05; *p<0.05; **p<0.005; ***p<0.001). Red dashed line indicates detection limit.

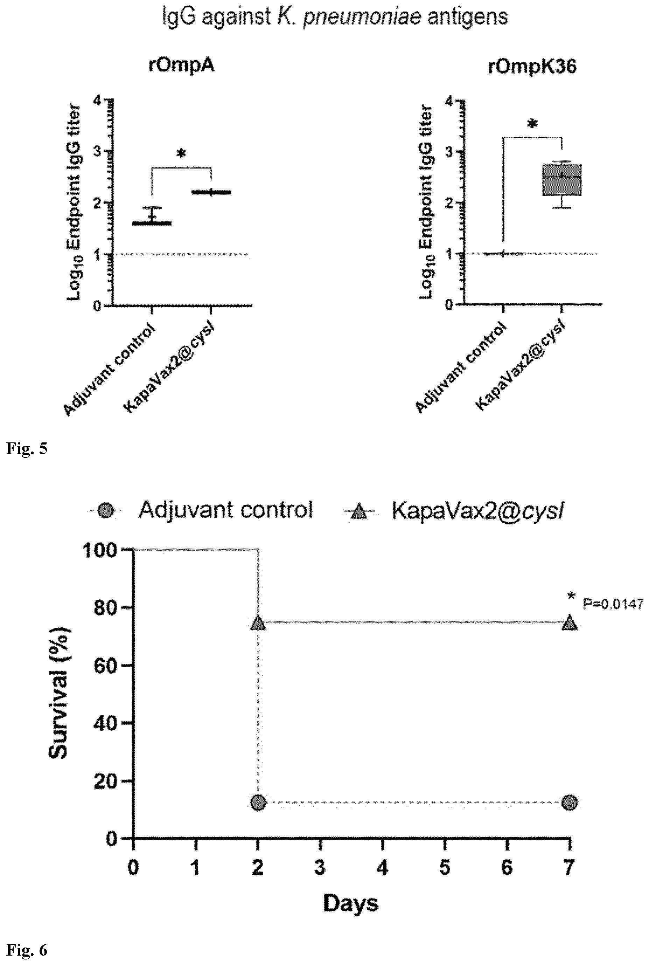

**Fig 5** shows IgG levels against *K. pneumoniae* antigens. ELISA recognition of recombinant proteins KpOmpA and KpOmpK36 by antisera from animals immunized with KapaVax2 (N=4) or vehicle alone (N=4), sampled at day 21 (7 days after 2nd immunization). Statistical analysis by Unpaired 2- tailed T- test (ns p>0,05; *p<0.05; **p<0.005; ***p<0.001). Red dashed line indicates detection limit.
**Fig. 6** shows a survival plot of sepsis model by *P. aeruginosa* infection. Animals were immunized with 2 doses in alternate weeks of the vaccine candidate KapaVax2, or vehicle alone. IP challenge with the strain PA14 (4.4x10⁶ cfu) was carried out 7 days after second immunization. Survival was monitored for 7 days. N/group= 8. Statistical differences of the survival curves are measured by Log-rank (Mantel-Cox) test. P. aeruginosa PA14 is a virulent wound-infection clinical isolate

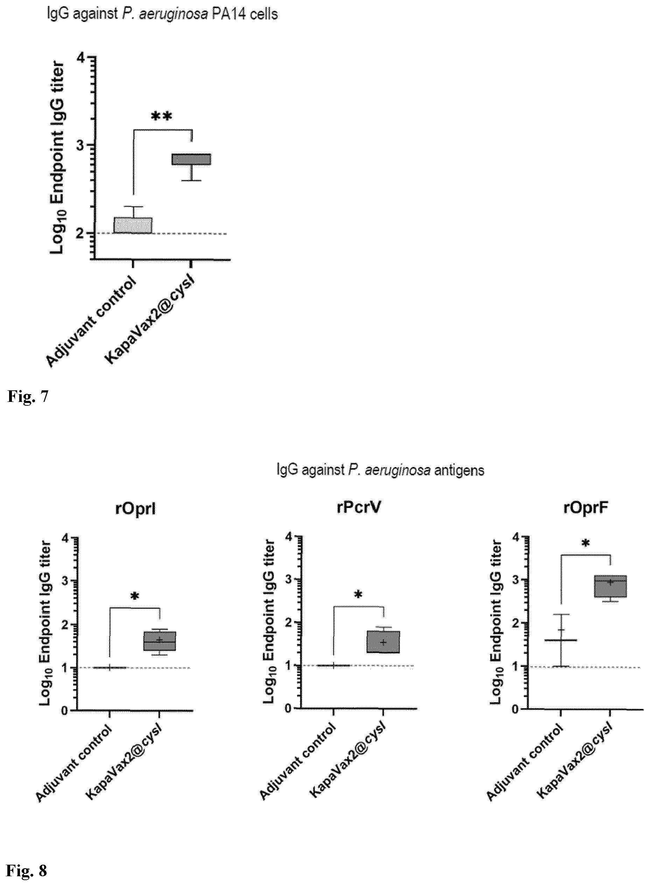

**Fig. 7** shows IgG levels against *P*. *aeruginosa* cells. ELISA recognition of *P. aeruginosa* PA14 cells by antisera from animals immunized with KapaVax2 (N=5) or vehicle alone (N=5), sampled at day 21 (7 days after 2nd immunization). Statistical significance: ns p>0,05; *p<0.05; **p<0.005; ***p<0.001. Red dashed line indicates detection limit.
**Fig. 8** shows IgG levels against *P. aeruginosa* antigens. ELISA recognition of recombinant proteins by antisera from animals immunized with 2 doses of KapaVax2 (N=4) or vehicle alone (N=4), sampled at day 21 (7 days after 2nd immunization). Statistical significance: ns p>0,05; *p<0.05; **p<0.005; ***p<0.001. Red dashed line indicates detection limit.

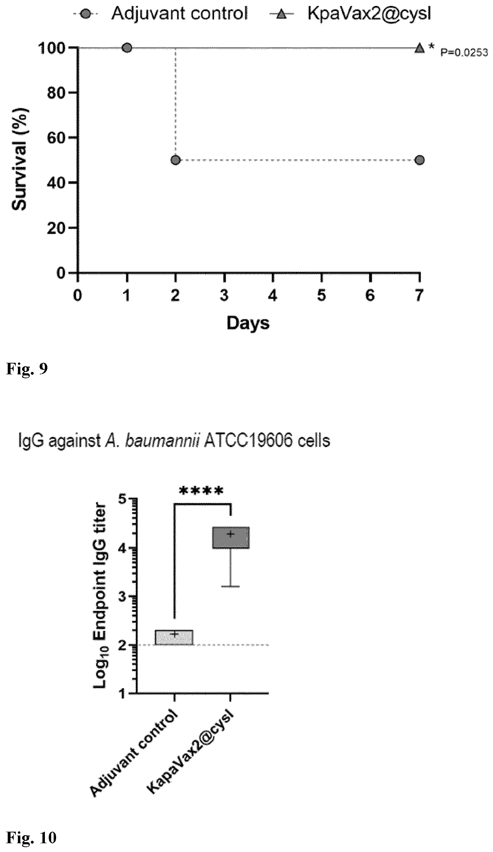

**Fig. 9****.** Shows survival plot of sepsis model by *A. baumannii.* Animals (N=8 per group) were immunized with 2 doses in alternate weeks of KapaVax2 or vehicle. IP challenge at day 7 after 2nd immunization, with 4.2x10⁶ cells of ATCC19606. Survival was monitored for 7 days. *A*. *baumannii* ATCC19606 is a urine infection clinical isolate used as reference strain in sepsis and pneumonia models of infection in mice.
**Fig. 10** shows IgG levels against *A. baumannii* cells. ELISA recognition of *A. baumannii* ATCC19606 cells by antisera from animals immunized with 2 doses of KapaVax2 (N=9) or vehicle alone (N=9), sampled at day 21 (7 days after 2nd immunization). Boxes show IQR, horizontal line and cross show median and mean, respectively. Error bars extend to the CI95%. Statistical analysis by Unpaired 2-tailed Mann-Whitney test (ns p>0,05; *p<0.05; **p<0.005; ***p<0.001). Red dashed line indicates detection limit.

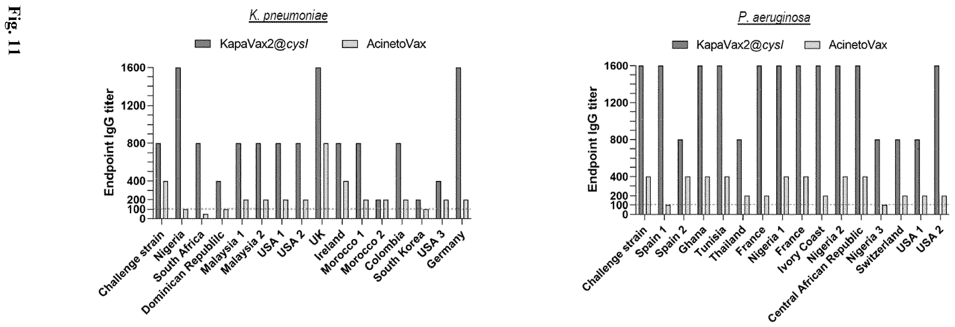

**Fig. 11** shows ELISA IgG titers (1:dilution) of detection of 15 clinical isolates strains of K. pneumoniae (9/15 LMICs), P. aeruginosa (8/15 LMICs) by antisera from KapaVax2 (LPS-deficient *A. baumannii* cells edited for expression of *K. pneumoniae* OmpA and OpmK36 and *P. aeruginosa* OprF and fusion protein OprI::PcrV and AcinetoVax (LPS-deficient *A. baumannii* cells, similar to the carrier cells edited for expression of the heterologous antigens in KapaVax)

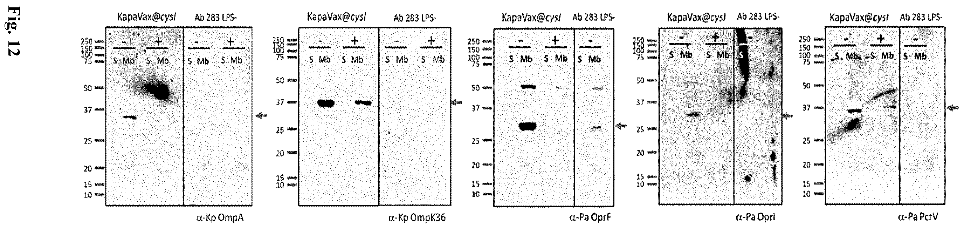

**Fig. 12** Surface exposure of heterologous antigens. Cultures of KapaVax (LPS-deficient A. baumannii cells edited for expression of *K. pneumoniae* OmpA and OpmK36 and *P. aeruginosa* OprF and fusion protein OprI::PcrV) were washed after growth, resuspended in PBS and treated with 0.5 mg/ml of Proteinase K for 1h at 37°C. Western-Blot with polyclonal anti-sera against recombinant proteins Kp-OmpA, Kp-OmpK36, Pa-OprF, Pa-OprI and Pa-PcrV. Note that both anti-Pa-OprI and anti-Pa-PcrV recognize the same fusion protein OprI::PcrV expressed in KapaVax2. Treatment with Proteinase K is indicated by + (treated) or - (untreated) in each sample). Mb: Outer-Membrane extract; S: extract supernatant. Protocol obtained from Richard N. Besingi and Patricia L. Clark. Nature Protocols. 2015 Dec; 10(12):2074-2080.

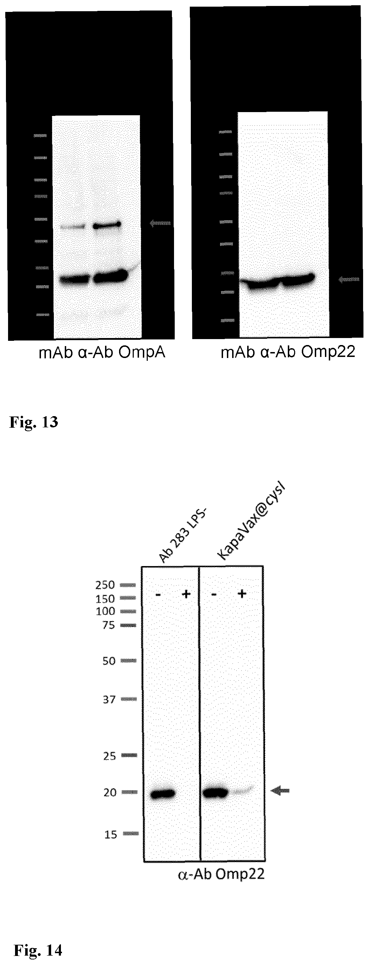

**Fig. 13** shows Expression of *A. baumannii* antigens in KapaVax and AcinetoVax vaccine batches: total lysate preparations from vaccine batches (2x1010 cells/ml). Samples were run on gels with 4- 16% acrylamide gradient. Panels show the signal obtained with monoclonal antibodies raised against OmpA (left) and Omp22 (right).
**Fig. 14** shows Surface exposure of *A*. *baumannii* Omp22 at the OM of KapaVax and the carrier cell Ab283 LPS-. Cultures were washed after growth, resuspended in PBS and treated with 0.5 mg/ml of Proteinase K for 1h at 37°C. Western-Blot with a monoclonal antibody raised against Ab-Omp22. Treatment with Proteinase K is indicated by + (treated) or - (untreated) in each sample). Mb: Outer-Membrane extract; S: extract supernatant. Protocol obtained from Richard N. Besingi and Patricia L. Clark. Nature Protocols. 2015 Dec; 10(12):2074-2080.

### Description of embodiments

The following detailed description discloses specific and/or preferred variants of the individual features of the invention. The present invention also contemplates as particularly preferred embodiments those embodiments, which are generated by combining two or more of the specific and/or preferred variants described for two or more of the features of the present invention. Unless expressly specified otherwise, the term "comprising" is used in the context of the present document to indicate that further members may optionally be present in addition to the members of the list introduced by "comprising".

The present invention is directed to:
a. an *A. baumannii* strain deficient in lipopolysaccharide (LPS) characterized by the partial or complete inactivation of one or various cellular nucleic acid molecules that encode endogenous LPS biosynthesis genes; wherein the *A. baumannii* strain deficient in lipopolysaccharide (LPS) is characterized by the partial or complete inactivation of the genes selected from the list consisting of *lpxA, lpxB, lpxC, lpxD, lpxK, lpxL* and/or *lpxM;* wherein the *A. baumannii* strain deficient in lipopolysaccharide (LPS) expresses one or more heterologous antigens with targeted location at the Outer Membrane, so that there is a surface exposure of such heterologous antigens; or
b. an outer membrane vesicle (OMV) derived from an *A. baumannii* strain deficient in lipopolysaccharide (LPS) as defined in the paragraph above.

In particular and as shown in the examples of the present specification, the expression of *K. pneumoniae* antigens (OmpA and OmpK36) and *P*. *aeruginosa* antigens (OprF, OprI and PcrV) on the outer-membrane of the Acinetobacter baumannii strain deficient in lipopolysaccharide (LPS), produces a trivalent preventive vaccine candidate efficient against *P. aeruginosa, K. pneumoniae,* and *A. baumannii* infection. The results obtained with such modififed strain provided herein support that immunity raised by the heterologous antigens was able to fully neutralize infection by a hypervirulent, hypercapsulated strain *K. pneumoniae* ATCC43816 (see figure 3); in addition, such strain led to significant protection against lethal sepsis produced by *P. aeruginosa* PA14 (see figure 6) and led to significant protection against lethal sepsis produced by *A. baumannii* ATCC19606 (see figure 9).

Therefore, the authors of the present invention demonstrate that, preferably inactivated, whole cells of *A. baumannii* deficient in LPS expressing one or multiple copies of antigenic outer-membrane heterologous proteins from one or more microorganisms, upon being inoculated in a subject, preferably a human subject, in need thereof, produce immunization not only against A. baumannii infection but also against infections caused by any microorganisms originally expressing the one or multiple copies of the antigenic outer-membrane proteins, which demonstrates the utility of these cells as prophylactic multivalent vaccines.

Consequently, a first aspect of the present invention refers to:
a. an *A. baumannii* strain deficient in lipopolysaccharide (LPS) characterized by the partial or complete inactivation of one or various cellular nucleic acid molecules that encode endogenous LPS biosynthesis genes; wherein the *A. baumannii* strain deficient in lipopolysaccharide (LPS) is characterized by the partial or complete inactivation of the genes selected from the list consisting of *lpxA, lpxB, lpxC, lpxD, lpxK, lpxL* and/or *lpxM*; and wherein the *A. baumannii* strain deficient in lipopolysaccharide (LPS) expresses one or multiple copies of antigenic outer-membrane heterologous proteins from one or more microorganisms with targeted location at the Outer Membrane; and/or
b. an outer membrane vesicle (OMV) derived from an *A. baumannii* strain deficient in lipopolysaccharide (LPS) as defined in the paragraph above.

It is noted that the ORFs of the one or multiple copies of antigenic outer-membrane heterologous proteins preferably comprises signal sequences promoting the location of the expressed proteins at the outer-membrane of the *A. baumannii* cells. The nature of these sequences are well-known in the art. In a preferred embodiment of this invention, the signal sequence of the outer-membrane protein OmpA of *A. baumannii* is used to promote location of the encoded proteins at the outer-membrane of *A. baumannii.*

It is understood that "cells of the *A. baumannii"* in the present invention are those cells pertaining to the domain Bacteria, phylum Proteobacteria, class Gammaproteobacteria, order Pseudomonadales, family *Moraxellaceae,* genus *Acinetobacer,* species *Acinetobacter baumannii.* The species of *Acinetobacter* are strictly aerobic non-fermenting and non-motile bacilli that are oxidase negative and appear in pairs by microscopy. They are distributed widely in nature, and are important in soil and contribute to its mineralization.

It is understood that the *A. baumannii* strains deficient in lipopolysaccharide (LPS) of the present invention are preferably whole inactivated cells, whole "inactivated cells" in the present invention are cells that do not have the ability to replicate but conserve their immunogenic capacity. The cells of the present invention are inactivated prior to their inoculation to prevent their replication in the host, and therefore prevent invention produced by their administration. The inactivation of the cells of the invention can be performed using diverse methods known in the state of the art for example, although not limited to, adsorption, heat, ultraviolet light, ionizing radiation, ultrasound, phenol, formol, formaldehyde, crystal violet, glyceraldehyde, ethylene oxide, propiolactone, ethylenamina, bromoethyleneamina or formalin. In a preferred form the cells of the invention are inactivated with heat. In another preferred form the cells of the invention are from the species Acinetobacter baumannii and they are inactivated with heat.

In a preferred embodiment of this aspect of the invention, the deficiency in LPS, as taught in EP2942389A1, can be achieved by partial or compete inactivation of one or various cellular molecules of nucleic acids that encode the endogenous genes for the LPS subunits, particularly *lpxA, lpxB* and/or *lpxC* of LPS. The sequences of *lpxA, lpxB* and/or *lpxC* from *Acinetobacter* species, particularly *A*. *baumannii.* In a preferred embodiment of the invention, the endogenous genes of LPS are selected from *lpxA, lpxB* and/or *lpxC,* or any combination of these genes.

In another preferred embodiment of the invention, the cell of *Acinetobacter* deficient in LPS is obtained by deletions and/or insertions of one or various nucleotides in nucleic acid sequences encoding the gene involved in the biosynthesis of LPS and/or the sequences that control their expression. The deletions and/or insertions can be generated by homologous recombination, insertion of transposons, or other adequate methods known in the state of the art.

In preferred embodiment of the invention, the sequence is inactivated e.g by construction of a suicide vector that contains the gene *lpxA, lpxB, lpxC, lpxD, lpxK, lpxL* and/or *lpxM* or any of their combination, or interrupting with a marker gene for selection, transforming the target cells with the vector and screening for positive cells that are negative for LPS expression.

In another preferred embodiment of the invention, although the cell of the invention is preferably an A. baumannii cell, such cell can be potentially replaced by other *Acinetobacter species* Acinetobacters species such as those selected from the list consisting of; *Acinetobacter baylyi, A. beijerinckii, A. bereziniae, A. boissieri, A. bouvetii, A. brisouii, A. calcoaceticus, A.gerneri, A. guillouiae, A. grimontii, A. gyllenbergii, A. haemolyticus, A. indicus, A. johnsonii, A. junii, A.Iwoffii, A. nectaris, A. nosocomialis, A. parvus, A. pittii, A. puyangensis, A. radioresistens, A. rudis, A. schindleri, A. soli, A. tandoii, A. tjernbergiae, A. towneri, A. ursingii or A. venetianus.* In this invention, it is understood that *Acinetobacter* refers to the kingdom Bacteria, phylum Proteobacteria, class Gammaproteobacteria, order Pseudomonadales, family *Moraxellaceae.*

It is understood that "lipopolysaccharide (LPS) or lipooligosaccharide" is a component that is found on the external membrane of various Gram-negative bacteria. The term LPS is used often and interchangeably with "endotoxin", due to its history of discovery. LPS consists of a polysaccharide chain and the rest is lipid, known as lipid A, which is responsible for the endotoxin activity. The polysaccharide chain is variable between different bacterias and determines the serotype. Endotoxin is of approximately 10 kDa in size, but can form large aggregates of up to 1000 kDa. Humans are able to produce antibodies against LPS, but in general these antibodies can only protect against bacteria of a specific serotype. Endotoxin is responsible for many of the clinical manifestations of infections caused by Gram-negative bacteria such as *Neisseria meningitidis* and *A. baumannii.*

As reflected above, the *A*. *baumannii* strain deficient in lipopolysaccharide (LPS) must express one or multiple copies of antigenic outer-membrane heterologous proteins from one or more microorganisms, with targeted location at its Outer Membrane. Such expression shall be carried out so that there is a surface exposure of such heterologous antigens. For that purpose, vaccine candidates of the present invention can be constructed by genome editing of *A. baumannii* using any allelic exchange technology for recombinant strain production that involves one or more recombination steps for insertion of an expression construct at a particular locus into the *A. baumannii* genome. The expression construct to be inserted could be carried into the cell by a linear piece of DNA or a plasmid. In particular, the construct coding for the one or multiple copies of the antigenic outer-membrane heterologous proteins can be inserted in any suitable locus of an *A. baumannii* strain previously, simultaneously or subsequently to the cell becoming deficient in LPS, as for example taught in EP2942389A1. A suitable locus is any locus on the *A. baumannii* chromosome that can incorporate an insert by recombination, such as: *cysI, trpE, lpxA, lpxC, lpxD, lpxB, lpxK, lpxL, lpxM,* and/or Tn5/Tn7 sites. It is noted that, Acinetobacter baumannii cells can be preferably transformed with a vector, preferably a suicide vector, comprising sequences for promoting recombination at the aimed target locus into the *A. baumannii* genome, being such locus any locus of the *A. baumannii* genome comprising sequences suitable to undergo recombination as detailed above.

The sequences of the vector for promoting recombination are flanking the expression construct comprising at least one or more transcription promoter sequences, one or more ORFs encoding proteins heterologous for *A. baumannii,* and one or more transcription termination sequences. The promoter sequence might be any known sequence in the state of the art able to promote transcription in an *A. baumannii* cell. These promoter sequences are routinely used in bacteriology research. In a prefered embodiment of this invention, the promoter sequence used is a promoter sequence located upstream of the *A. baumannii* ORF encoding the outer-membrane protein OmpA. The expression construct might comprise several ORFs in tandem, each one flanked by a promoter and a termination, transcriptional termination, sequence upstream and downstream of the ORF, respectively, therefore built for allowing independent expression of each ORF, controlled by a specific promoter. Alternatively, the expression construct might have one or several ORFs within an operon-like structure with polycistronic expression controlled by a common promoter. The ORFs of the expression vector encode proteins or peptides selected by their immunogenic properties, derived from known humans or animal pathogens distinct from *A. baumannii* and whose expression in the *A. baumannii* cell is intended for raising an immune response against pathogens distinct from *A. baumannii* in a human or animal vaccinated with the *A. baumannii* cell. The ORFs of the expression construct might encode for any peptide with immunogenic properties derived from any known pathogen. In a preferred embodiment of this invention, the ORFs of the expression construct encode peptides derived from the Gram-negative bacteria *K. pneumoniae* and/or *P. aeruginosa.* In another prefered embodiment of this invention, the peptides derived from K. pneumoniae comprise sequences derived from the outer-membrane proteins OmpA and/or OmpK36 from *K. pneumoniae.* In another prefered embodiment of this invention, the peptides derived from *P. aeruginosa* comprise sequences derived from the outer-membrane proteins OprF and/or OprI from *P. aeruginosa* and/or the protein PcrV of the Type 3 secretion system from *P. aeruginosa.* The ORF sequences might include sequences for assisting post-translational processing of the encoded proteins. In particular, the ORFs might contain signal sequences promoting the location of the expressed proteins at the outer-membrane of the *A. baumannii* cells. The nature of these sequences are well-known in the art. In a preferred embodiment of this invention, the signal sequence of the outer-membrane protein OmpA of *A. baumannii* is used to promote location of the encoded proteins at the outer-membrane of *A. baumannii.* The transcription termination sequences of the expression construct might be any transcription termination sequence able to allow transcription termination in *A. baumannii,* well known in the art.

Second, as explained below in the specification, once the *A. baumannii* cells are transformed with the vector, preferably a suicide vector, recombination between the sequences of the target loci in the host and the sequences in the vector leads to the production of recombinant cells where the sequences of the vector have integrated into the targeted loci on the A. baumannii chromosome. This recombination event can be assisted by strategies well-known in the art, like inducing double strand breaks at the insertion site with genome-editing tools, like those based on the CRISPR methodology. In a preferred embodiment of this invention, a first recombination event leads to the insertion of the vector sequences comprising the selectable markers and the expression contructs. The selectable markers are used for selection of cells where such first recombination event has ocurred. A second recombination event might lead to recombinant cells where only the expression construct but not the sequences encoding the selectable markers remain integrated at the insertion site. By using as a selection tool the lack of the selectable markes, *A. baumannii* cells where such second recombination event has ocurred can be selected. Once selected, common techniques like PCR and DNA sequencing are used to check whether the integration of the vector sequences into the targeted loci has ocurred as expected.

On the other hand, Prior to, simulaltenously to or subsequently to the expression of the heterologous proteins analyzed in the recombinant *A. baumannii* cells is considered satisfactory, loss of the LPS is induced in the *A. baumannii* cells by alternative methods like site-directed mutagenesis of a LPS-synthesis gene leading to partial or complete inactivation of LPS-synthesis genes, including the genes selected from the list consisting *lpxA, lpxB, lpxC, lpxD, lpxK, lpxL* and/or *lpxM*; or by selecting *A. baumannii* cells resistant to the antibiotic colistin and screening the cells by common genomic methods like PCR and/or DNA sequencing for mutations leading to partial or complete inactivation of LPS-syntheiss genes, including the genes selected from the list consisting of *lpxA, lpxB, lpxC, lpxD, lpxK, lpxL* and/or *lpxM.*

In a particular embodiment of this invention, when the targeted locus for insertion of the recombination events described above is a locus comprising an ORF encoding an LPS-synthesis gene, including genes selected from the list consisting of *lpxA, lpxB, lpxC, lpxD, lpxK, lpxL* and/or *lpxM,* and the recombination leads to partial or complete inactivation of such ORF, the resulting recombinant *A. baumannii* cells are LPS-negative cells due to partial or complete inactivation of LPS-syntheiss genes, including the genes selected from the list consisting *lpxA, lpxB, lpxC, lpxD, lpxK, lpxL* and/or *lpxM.*

After having inserted the constructs at the suitable locus, such as at the *cysI* locus, proof of expression and localization of the heterologous antigens at the outer-membrane can be performed (as taught in the examples), then proof of selection of an LPS-negative derivative can be carried out for example by plating in colistin and selection of an LPS-negative mutant. Expression and location at the outer membrane of each antigen can be confirmed by Western Blot and ELISA. Western blot analysis of whole cell lysates and outer membrane extracts with antigen-specific antibodies can be thus used to confirm expression and localization of the heterologous antigens in the the *A. baumannii* cell. Recognition of recombinant antigens on the surface of whole cells can also be verified using antigen- specific antibodies in ELISA experiments.

In a particular embodiment of the first aspect of the invention, the heterologous antigens expressed at the Outer Membrane of the *A. baumannii* strain deficient in lipopolysaccharide (LPS) are at least derived from *K. pneumoniae* and/or *P. aeruginosa.* In particular, the heterologous antigens expressed at the Outer Membrane of the *A*. *baumannii* strain deficient in lipopolysaccharide (LPS) are at least derived from *K. pneumoniae* and are selected from the list consisting of Kp-OmpA (SEQ ID No, 1) and Kp-Ompk36 (SEQ ID No. 2).

**-OmpA (K1).** OmpA is a highly conserved membrane porin among the *Enterobacteriaceae.* It is not only the homologous version of one of the 2 major antigens identified as responsible for protection exerted by our LPS-null *A. baumannii* cells in animal models, but it has been described as the protein antigen most recognized by antisera from patients with acute infections produced by *K. pneumoniae* (1). Moreover, its use as a DNA vaccine (2) led to protective immune responses in murine models of sepsis. Briefly, results published by Kurupati et al (2) show that intramuscular immunization of BALB/c females with 4 doses of 50 ul of ompA DNA preparations increases survival at 8 days up to 60% against a challenge with a lethal dose of *K. pneumoniae* clinical isolate.

**-OmpK36 (K2).** OmpK36, also a highly conserved membrane porin in *K. pneumoniae,* has been reported as the second (after OmpA) most recognized protein by antisera from infected patients, with the highest coverage with sera from different clinical infections (1). Its use as a DNA vaccine in a murine sepsis model described above (2), showed even higher levels of protection than those obtained by vaccination with OmpA (2), specifically survival rate at 8 days increases up to 75%, versus 60% obtained with a vaccine expressing only OmpA. In addition, when used as a single subunit vaccine, immunization with 3 doses of 25 ug of full-length recombinant OmpK36 in combination with incomplete Freund's adjuvant was able to protect mice from intraperitoneal challenge (survival rate of 60%), and immune sera were able to neutralize diverse strains of *K. pneumoniae* (3)

In another particular embodiment of the first aspect of the invention, the heterologous antigens expressed at the Outer Membrane of the *Acinetobacter baumannii* strain deficient in lipopolysaccharide (LPS) are at least derived from *P. aeruginosa* and are selected from the list consisting of Pa-OprF (SEQ ID NO. 3), Pa-OprI (SEQ ID NO. 5), Pa- PcrV (SEQ ID NO 6), or Pa-OprI:PcrV (SEQ ID NO 4).

**-OprI (P1).** OprI is a highy conserved membrane lipoprotein that has been demonstrated to exert immunostimuatory activites via activation of Toll-like Receptors (TLRs) 2 y 4, an important step in the indiution of Thl type T cells, when used as adjuvant in a protein based vaccine againts *Mycobacterium tuberculosis* consititing in a fusion protein N-terminal OrI-Ag85 of *M tuberculosis* (5). This Th1 inducing property of OprI was previously demonstrated in a mouse model of cutaneous leishmaniasis (6) and in a model of classical swine feber (7). The protective effect of OprI agains *P. aeruginosa* infection was desmotrated in a murine model of sepsis using immunocompromised Balb/c females, where the LD50 was up to 35-fold higher in animals immunized intraperitoneally with 4 doses of 50 ug of purified OprI, versus animals not immnunized (8,9). Importantly, immunization with OprI was shown to be safe and immunogenic in healthy volunteers (10)

**-PcrV (P2).** PcrV is the major antigen of the needles formed by the *P. aeruginosa* Type 3 secretion system apparatus. It has been shown that anti-PcrV antibodies contribute significantly to protection against virulent *P*. *aeruginosa* infections (11-14), and that active vaccination with PcrV can induce protective immunity against *P*. *aeruginosa* infection (15,16). Results obtained by Meynet et al (23) show that subcutaneal immunization of C57B1/6J females with 3 doses at 2 week intervals containing 1-2x10⁸ CFU of killed but metabolic active *P. aeruginosa* overexpressing PcrV results in survival at 7 days of 58.3% ompared to unvaccinated animals against *in an* acute lung infection model. In addition, Hamaoka S. et al (2017) tested the effect of immunization with recombinant PcrV (rPcrV) in combination with three adyumvants in a murine model of acute pneumonia (17). Results of intraperitoneal immunization with 3 doses of 10 ug of rPcrV each, in combination with AlOH₃ increased survival to 73% compared to the control group immunizated with equivalent doses of adyuvant. Importantly, two monoclonal antibody-based therapies targeting PcrV are currently in clinical phases of development, with one study demonstrating a significant decrease in *P. aeruginosa* infection rates (pneumonia) compared to placebo (18,19).

**OprF (P3).** OprF is a highly-conserved OmpA homolog, and a major outer membrane porin in *P. aeruginosa.* Previous immunization studies in experimental models have shown that OprF protects against *P*. *aeruginosa* infections. Gilleland Jr, et al. (1984) demonstrated that intraperitoneal inmunization with 2 doses of 10 ug purified OprF from *P. aeruginosa* PAO1 protects up to 67% in a murine model of sepsis using CD-1 mice (20). Moreover, Hassan et al. have recently reported immunization of mice with recombinant full-length OprF from *P. aeruginosa* obtained by heterologous expression in *Escherichia coli* under the control of an inducible promoter (21). The full-length OprF used as a subunit vaccine in a murine model of acute pneumonia was able to protect Balb/c female mice from infection by non-mucoid and mucoid *P. aeruginosa* strains PAO1 and PAK, respectively. While 100% of non immunized animals died 48-72 after intranasal inoculation with *P. aeruginosa,* survival at 7 days of animals immunized subcutaneously with 4 doses recombinant OprF (rOprF), containing 50ug of recombinant protein, was 50 and 60% for PaO1 and PAK respectively. Nevertheless, in the same study, survival against PAO1 challenge is improved to 100% when the rOprF is combined with recombinant OprI (21).
Sequence ID Number 1. *K. pneumoniae* OmpA 
**atgaaattgagtcgtattgcacttgctactatgcttgttgctgctccattagctgctgctaatgct**ggtggtaaactgggttggtccc
agtatcacgacaccggtttctacggtaacggtttccagaacaacaacggtccgacccgtaacgatcagcttggtgctggtgcgttcggt
ggttaccaggttaacccgtacctcggtttcgaaatgggttatgactggctgggccgtatggcatataaaggcagcgttgacaacggtgc
tttcaaagctcagggcgttcagctgaccgctaaactgggttacccgatcactgacgatctggacatctacacccgtctgggcggcatgg
tttggcgcgctgactccaaaggcaactacgcttctaccggcgtttcccgtagcgaacacgacactggcgtttccccagtatttgctggc
ggcgtagagtgggctgttactcgtgacatcgctacccgtctggaataccagtgggttaacaacatcggcgacgcgggcactgtgggt
 
acccgtcctgataacggcatgctgagcctgggcgtttcctaccgcttcggtcaggaagatgctgcaccggttgttgctccggctccggc
tccggctccggaagtggctaccaagcacttcaccctgaagtctgacgttctgttcaacttcaacaaagctaccctgaaaccggaaggtc
agcaggctctggatcagctgtacactcagctgagcaacatggaCccgaaagacggttccgctgttgttctgggctacaccgaccgca
tcggttccgaagcttacaaccagcagctgtctgagaaacgtgctcagtccgttgttgactacctggttgctaaaggcatcccggctggc
aaaatctccgctcgcggcatgggtgaatccaacccggttactggcaacacctgtgacaacgtgaaagctcgcgctgccctgatcgatt
gcctggctccggatcgtcgtgtagagatcgaagttaaaggctacaaagaagttgtaactcagccgcaggcttaa
 
KLSRIALATMLVAAPLAAANAGGKLGWSQYHDTGFYGNGFQNNNGPTRNDQLGA
GAFGGYQVNPYLGFEMGYDWLGRMAYKGSVDNGAFKAQGVQLTAKLGYPITDDL
DIYTRLGGMVWRADSKGNYASTGVSRSEHDTGVSPVFAGGVEWAVTRDIATRLEY
QWVNNIGDAGTVGTRPDNGMLSLGVSYRFGQEDAAPVVAPAPAPAPEVATKHFTL
KSDVLFNFNKATLKPEGQQALDQLYTQLSNMDPKDGSAVVLGYTDRIGSEAYNQQL
SEKRAQSVVDYLVAKGIPAGKISARGMGESNPVTGNTCDNVKARAALIDCLAPDRR
VEIEVKGYKEVVTQPQA
 ***In bold *A. baumannii* signal peptide**
Sequence ID Number 2. *K. pneumoniae* OmpK36 
atgaaattgagtcgtattgcacttgctactatgcttgttgctgctccattagctgctgctaatgctgctgaaatttataacaaagacg
gcaacaaattagacctgtacggtaaaattgacggtctgcactacttctctgacgacaagagcgtcgacggcgaccagacctacatgcg
tgtaggcgtgaaaggcgaaacccagatcaacgaccagctgaccggttacggccagtgggaatacaacgttcaggcgaacaacactg
aaagctccagcgatcaggcatggactcgtctggcattcgcaggcctgaaatttggcgacgcgggctctttcgactacggtcgtaacta
cggcgtagtatacgacgtaacgtcctggaccgacgttctgccggaattcggcggcgacacctacggttctgacaacttcctgcagtcc
cgtgctaacggcgttgcaacctaccgtaactctgatttcttcggtctggttgacggcctgaactttgctctgcagtatcagggtaaaaacg
gcagcgtcagcggcgaaggcgctctgtctcctaccaacaacggtcgtaccgccttgaaacagaacggcgacggttacggtacttctc
tgacctatgacatctatgatggcatcagcgctggtttcgcatactctaactccaaacgtcttggcgaccagaacagcaagctggcactg
ggtcgtggcgacaacgctgaaacctacaccggcggtctgaaatacgacgcgaacaacatctacctggccactcagtacacccagac
ctacaacgcgacccgcgccggttccctgggctttgctaacaaagcgcagaacttcgaagtggttgctcagtaccagttcgacttcggtc
tgcgtccgtccgtggcttacctgcagtctaaaggtaaggatctggaaggctacggcgaccaggacatcctgaaatatgttgacgttggc
gcgacctactacttcaacaaaaacatgtccacctatgttgactacaaaatcaacctgctggacgacaatagcttcacccacaacgccgg
tatctctaccgacgacgtggttgcactgggcctggtttaccagttctaa
 
MKLSRIALATMLVAAPLAAANAAEIYNKDGNKLDLYGKIDGLHYFSDDKSVDGD
QTYMRVGVKGETQINDQLTGYGQWEYNVQANNTESSSDQAWTRLAFAGLKFGDA
GSFDYGRNYGVVYDVTSWTDVLPEFGGDTYGSDNFLQSRANGVATYRNSDFFGLV
 
DGLNFALQYQGKNGSVSGEGALSPTNNGRTALKQNGDGYGTSLTYDIYDGISAGFA
YSNSKRLGDQNSKLALGRGDNAETYTGGLKYDANNIYLATQYTQTYNATRAGSLG
FANKAQNFEVVAQYQFDFGLRPSVAYLQSKGKDLEGYGDQDILKYVDVGATYYFN
KNMSTYVDYKINLLDDNSFTHNAGISTDDVVALGLVYQF
*In bold A. baumannii signal peptide
Sequence ID Number 3. *P. aeruginosa* OprF 
atgaaattgagtcgtattgcacttgctactatgcttgttgctgctccattagctgctgctaatgctcagggccagaactcggtagag
atcgaagccttcggcaagcgctacttcaccgacagcgttcgcaacatgaagaacgcggacctgtacggcggctcgatcggttacttcc
tgaccgacgacgtcgagctggcgctgtcctacggtgagtaccatgacgttcgtggcacctacgaaaccggcaacaagaaggtccac
ggcaacctgacctccctggacgccatctaccacttcggtaccccgggcgtaggtctgcgtccgtacgtgtcggctggtctggctcacc
agaacatcaccaacatcaacagcgacagccaaggccgtcagcagatgaccatggccaacatcggcgctggtctgaagtactacttca
ccgagaacttcttcgccaaggccagcctcgacggccagtacggtctggagaagcgtgacaacggtcaccagggcgagtggatggc
tggcctgggcgtcggcttcaacttcggtggttcgaaagccgctccggctccggaaccggttgccgacgtttgctccgactccgacaac
gacggcgtttgcgacaacgtcgacaagtgcccggataccccggccaacgtcaccgttgacgccaacggctgcccggctgtcgccg
aagtcgtacgcgtacagctggacgtgaagttcgacttcgacaagtccaaggtcaaagagaacagctacgctgacatcaagaacctgg
ctgacttcatgaagcagtacccgtccacttccaccaccgttgaaggtcacaccgactccgtcggcaccgacgcttacaaccagaagct
gtccgagcgtcgtgccaacgccgttcgtgacgtactggtcaacgagtacggtgtagaaggtggtcgcgtgaacgctgttggttacggc
gagtcccgcccggttgccgacaacgccaccgctgaaggccgcgctatcaaccgtcgcgttgaagccgaagtagaagctgaagcca
agtaa
 
MKLSRIALATMLVAAPLAAANAQGQNSVEIEAFGKRYFTDSVRNMKNADLYGGSI
GYFLTDDVELALSYGEYHDVRGTYETGNKKVHGNLTSLDAIYHFGTPGVGLRPYVS
AGLAHQNITNINSDSQGRQQMTMANIGAGLKYYFTENFFAKASLDGQYGLEKRDNG
HQGEWMAGLGVGFNFGGSKAAPAPEPVADVCSDSDNDGVCDNVDKCPDTPANVT
VDANGCPAVAEVVRVQLDVKFDFDKSKVKENSYADIKNLADFMKQYPSTSTTVEG
HTDSVGTDAYNQKLSERRANAVRDVLVNEYGVEGGRVNAVGYGESRPVADNATA
EGRAINRRVEAEVEAEAK
Sequence ID Number 4. *P. aeruginosa* OprI::PcrV fusion protein 
atgaaattgagtcgtattgcacttgctactatgcttgttgctgctccattagctgctgctaatgctagccactccaaagaaaccgaa
gctcgtctgaccgctaccgaagacgcagctgctcgtgctcaggctcgcgctgacgaagcctatcgcaaggctgacgaagctctggg
cgctgctcagaaagctcagcagactgctgacgaggctaacgagcgtgccctgcgcatgctggaaaaagccagccgcaagggcgg
 
cggcggcagcggcggcggcggcagcggcggcggcagctcggcggcgcctgccagtgccgagcaggaggaactgctggc
cctgttgcgcagcgagcggatcgtgctggcccacgccggccagccgctgagcgaggcgcaagtgctcaaggcgctcgcctggttg
ctcgcggccaatccgtccgcgcctccggggcagggcctcgaggtactccgcgaagtcctgcaggcacgtcggcagcccggtgcg
cagtgggatctgcgcgagttcctggtgtcggcctatttcagcctgcacgggcgtctcgacgaggatgtcatcggtgtctacaaggatgt
cctgcagacccaggacggcaagcgcaaggcgctgctcgacgagctgaaggcgctgaccgcggagttgaaggtctacagcgtgat
ccagtcgcagatcaacgccgcgctgtcggccaagcagggcatcaggatcgacgctggcggtatcgatctggtcgaccccacgctat
atggctatgccgtcggcgatcccaggtggaaggacagccccgagtatgcgctgctgagcaatctggataccttcagcggcaagctgt
cgatcaaggattttctcagcggctcgccgaagcagagcggggaactcaagggcctcagcgatgagtaccccttcgagaaggacaac
aacccggtcggcaatttcgccaccacggtgagcgaccgctcgcgtccgctgaacgacaaggtcaacgagaagaccaccctgctcaa
cgacaccagctcccgctacaactcggcggtcgaggcgctcaaccgcttcatccagaaatacgacagcgtcctgcgcgacattctcag
cgcgatctag
 
MKLSRIALATMLVAAPLAAANASHSKETEARLTATEDAAARAQARADEAYRKAD
EALGAAQKAQQTADEANERALRMLEKASRKGGGGSGGGGSGGGGSSAAPASAEQE
ELLALLRSERIVLAHAGQPLSEAQVLKALAWLLAANPSAPPGQGLEVLREVLQARRQ
PGAQWDLREFLVSAYFSLHGRLDEDVIGVYKDVLQTQDGKRKALLDELKALTAELK
VYSVIQSQINAALSAKQGIRIDAGGIDLVDPTLYGYAVGDPRWKDSPEYALLSNL
DTFSGKLSIKDFLSGSPKQSGELKGLSDEYPFEKDNNPVGNFATTVSDRSRPLND
KVNEKTTLLNDTSSRYNSAVEALNRFIQKYDSVLRDILSAI
 ***In bold *A. baumannii* signal peptide** *linker underlined
Sequence ID Number 5. *P. aeruginosa* OprI 
**atgaaattgagtcgtattgcacttgctactatgcttgttgctgctccattagctgctgctaatgct**agccactccaaagaaaccgaa
gctcgtctgaccgctaccgaagacgcagctgctcgtgctcaggctcgcgctgacgaagcctatcgcaaggctgacgaagctctggg
cgctgctcagaaagctcagcagactgctgacgaggctaacgagcgtgccctgcgcatgctggaaaaagccagccgcaatag
**MKLSRIALATMLVAAPLAAANA**SHSKETEARLTATEDAAARAQARADEAYRKAD
EALGAAQKAQQTADEANERALRMLEKASRK
 ***In bold *A. baumannii* signal peptide**
Sequence ID Number 6. *P. aeruginosa* PcrV

atggaagtcagaaaccttaatgccgctcgcgagctgttcctggacgagctcctggccgcgtcggcggcgcctgccagtgccgagca
ggaggaactgctggccctgttgcgcagcgagcggatcgtgctggcccacgccggccagccgctgagcgaggcgcaagtgctcaa
ggcgctcgcctggttgctcgcggccaatccgtccgcgcctccggggcagggcctcgaggtactccgcgaagtcctgcaggcacgtc
ggcagcccggtgcgcagtgggatctgcgcgagttcctggtgtcggcctatttcagcctgcacgggcgtctcgacgaggatgtcatcg
gtgtctacaaggatgtcctgcagacccaggacggcaagcgcaaggcgctgctcgacgagctcaaggcgctgaccgcggagttgaa
ggtctacagcgtgatccagtcgcagatcaacgccgcgctgtcggccaagcagggcatcaggatcgacgctggcggtatcgatctgg
tcgaccccacgctatatggctatgccgtcggcgatcccaggtggaaggacagccccgagtatgcgctgctgagcaatctggatacctt
cagcggcaagctgtcgatcaaggattttctcagcggctcgccgaagcagagcggggaactcaagggcctcagcgatgagtacccct
tcgagaaggacaacaacccggtcggcaatttcgccaccacggtgagcgaccgctcgcgtccgctgaacgacaaggtcaacgagaa
gaccaccctgctcaacgacaccagctcccgctacaactcggcggtcgaggcgctcaaccgcttcatccagaaatacgacagcgtcct
gcgcgacattctcagcgcgatctag

MEVRNLNAARELFLDELLAASAAPASAEQEELLALLRSERIVLAHAGQPLSEAQVLK
ALAWLLAANPSAPPGQGLEVLREVLQARRQPGAQWDLREFLVSAYFSLHGRLDEDV
IGVYKDVLQTQDGKRKALLDELKALTAELKVYSVIQSQINAALSAKQGIRIDAGGID
LVDPTLYGYAVGDPRWKDSPEYALLSNLDTFSGKLSIKDFLSGSPKQSGELKGLSDE
YPFEKDNNPVGNFATTVSDRSRPLNDKVNEKTTLLNDTSSRYNSAVEALNRFIQKYD
SVLRDILSAI

In another embodiment of the first aspect of the invention, the heterologous antigens expressed at the Outer Membrane of the *A. baumannii* strain deficient in lipopolysaccharide (LPS) are at least derived from *K. pneumoniae* and are selected from the list consisting of Pa-OprF and Pa-OprI:PcrV, and/or are derived from *P. aeruginosa* and are selected from the list consisting of Pa-OprF, Pa-OprI, Pa- PcrV, and preferably the *A. baumannii* strain deficient in lipopolysaccharide (LPS) further comprises the expression of *A. baumannii* antigens Ab-OmpA and Ab-Omp22.

In another embodiment of the first aspect of the invention, the *A. baumannii* strain deficient in lipopolysaccharide (LPS) is characterized by the partial or complete inactivation of the genes selected from the list consisting of *lpxA, lpxC,* and *lpxD.*

A second aspect of the invention refers to a composition, hereinafter composition of the invention, comprising the *A. baumannii* strain deficient in lipopolysaccharide (LPS) expressing the one or multiple copies of antigenic outer-membrane heterologous proteins from one or more microorganisms at its Outer Membrane, as defined in the first aspect of the invention or in any of its preferred embodiments.

Preferably, the composition of the invention, is a pharmaceutical composition optionally comprising acceptable pharmaceutical vehicles, carriers and/or excipients. Still more preferably, the composition of the invention is a vaccine formulation optionally comprising an adjuvant. Preferably the composition or the vaccine formulation, comprises from about 10⁶ to about 10¹² *A. baumannii* strains deficient in lipopolysaccharide (LPS) expressing the one or multiple copies of antigenic outer-membrane heterologous proteins from one or more microorganisms at its Outer Membrane, as defined in the first aspect of the invention or in any of its preferred embodiments, preferably about 10⁹ of such strains. Also preferably, the adjuvant, if present in the vaccine formualation, is preferably Al(OH)₃. Further preferably, the *A. baumannii* strains deficient in lipopolysaccharide (LPS) present in the composition, preferably in the vaccine formulation, are inactivated. Nothwithstanding the above, the dosing for obtaining an effective therapeutic quantity depends on a varitey of factors such as for example, age, weight, sex, tolerante,... of the mammal. As used in this description, any therapeutic quantity should be effective and should thus refer to the quantity of inactive cells of the genus *Acinetobacter* that produces the desired effect, and in general are determined by the therapeutic effect that is desired.

The term "excipient" makes reference to a substance that helps in the absorption of the elements of the composition of the medicaments of the invention, stabilizing said elements, activating or helping the preparation of the medicament such that it provides consistency or flavours that make it more palatable. The excipeints can maintain the ingredients together, like for example is the case with starches, sugars, cellulose, sweetners, coloring agents, the function of protecting the medicament, for example isolating it form air and/or humidity, the function of filling the pill, capsule or any other form of presentation, for example, the case of dibasic calcium phosphate, the function for facilitating dissolution of the components and their absorption in the intestine, without excluding other types of excipients described in this paragraph.

The vehicle, in the same way as the excipient, is a substance that is used in the medicament to dilute any of the components of the present invention to a desired volume or weight. The pharmaceutically acceptable vehicle is an inert substance or of similar action to any of the elements of the present invention. The function of the vehicle is to facilitate the incorporation of other elements, permit better dosing and administration and give consistency and form to the medicament. When the form of presentation is liquid, the pharmaceutically acceptable vehicle is the diluent.

The adjuvants and pharmaceutically acceptable vehicle that can be used in the composition of the invention are those vehicles known by experts in the field. In this invention, the term "adjuvant" refers to any agent that does not pose antigenic activity in and of itself, that can be used to stimulate the immune system to increase the response to a vaccine. There are many adjuvants, for example but not limited to, aluminium phosphate, aluminium hydroxide, toll-like receptor agonists, cytokines, squaline, Freunds incomplete and complete adjuvants. In a preferred form of this aspect of the invention, the adjuvant is selected for a list that consists of aluminium phosphate, aluminium hydroxide, toll-like receptor agonists, cytokines, squaline, saponins, Freunds incomplete and complete adjuvants.

In the context of the present invention, the term "vaccine" refers to an antigenic preparation employed for inducing an immune response to a disease. They are prepared from antigens that, once inside the host, provoke an immune response through the production of antibodies, and generate immunologic memory producing transient or permanent immunity.

Manufacturing of the vaccine formulation of the present invention can be achieved, but without being limited to, via a 2-step fermentation process and subsequent heat-inactivation. The vaccine formulation shall be preferably in the form of a sterile, lyophilized preparation of the vaccine. Lyophilization shall be applied to the final product, once it has been formulated with excipients and the adjuvant. The vaccine formulation based on the LPS-null *A. baumannii* cell technology is preferably not filtered or autoclaved in order to obtain sterility. The cells are usually inactivated in the last step of manufacturing of the drug substance after fermentation, before formulating the final product. The inactivation method shall be preferably based on a mild heat-inactivation protocol (75 °C for 30 min) in order to preserve the potency of the whole-cell, multi-antigen active ingredient.

A third aspect refers to the *A. baumannii* strain deficient in lipopolysaccharide (LPS) expressing the one or multiple copies of antigenic outer-membrane heterologous proteins from one or more microorganisms at its Outer Membrane, as defined in the first aspect of the invention or in any of its preferred embodiments or to the composition of the second aspect of the invention, for use as a medicament or for use in therapy. More preferably, for use as a vaccine for inducing an immune response to a disease caused by any microorganisms originally expressing the one or multiple copies of the antigenic Outer Membrane proteins.

The term "medicament" as used in this report, makes reference to any substance used for the prevention, alleviation, treatment or cure of a disease in man or animals.

In a fourth aspect, the *A. baumannii* strain deficient in lipopolysaccharide (LPS) of the first aspect of the invention or the composition of the second aspect of the invention, is use for delivering bacterial Outer Membrane antigens to a subject, preferably a human subject, in need thereof for immunizing said subject against any infectious disease comprising, carrying or expressing said antigens. In a preferred embodiment, the *A. baumannii* strain deficient in lipopolysaccharide (LPS) of the first aspect of the invention or the composition of the second aspect of the invention, is use for delivering bacterial Outer Membrane antigens for inducing an immunological response against *K. pneumoniae* and/or *P. aeruginosa* and optionally *A. baumannii* in a subject in need thereof. In a preferred embodiment, the *A. baumannii* strain deficient in lipopolysaccharide (LPS) of the first aspect of the invention or the composition of the second aspect of the invention, is use for delivering bacterial Outer Membrane antigens for the prevention, improvement or treatment of an infection caused by *K. pneumoniae* and/or *P. aeruginosa* and optionally *A*. *baumannii.* Preferably, for any of these purposes the heterologous antigens expressed at the Outer Membrane of the *A. baumannii* strain deficient in lipopolysaccharide (LPS) are at least derived from *K. pneumoniae* and are selected from the list consisting of Pa-OprF and/or Pa-OprI:PcrV, and/or are derived from *P. aeruginosa* and are selected from the list consisting of Pa-OprF, OprI and/or PcrV and preferably the *A*. *baumannii* strain deficient in lipopolysaccharide (LPS) further comprises the expression of *A. baumannii* antigens Ab-OmpA and/or Ab-Omp22.

The medicaments and compositions of the invention can be used alone or in combination with other medicaments or active ingredients or compositions for the treatment of diseases produced by organisms from the genus *Acinetobacter.*

As used here, the term "active ingredient" refers to any component that potentially provides pharmacological activity or other different effect in the diagnosis, cure, alleviation, treatment or prevention of a disease or that affects the structure or function of the human or animal body. The term includes those components that promote a chemical change in the elaboration of the drug and are present in the same and a modified form that provides specific activity or the effect.

It is understood by "an infection caused by *K. pneumoniae* and/or *P. aeruginosa* and optionally *A. baumannii"* those diseases in which the causal agent of the pathology is any of *K. pneumoniae* and/or *P. aeruginosa* and/or *A. baumannii.* The genus *Acinetobacter* produces diverse pathologies for example but not limited to, bacteremia, meningitis, urinary tract infections, skin and soft tissue infections, surgical site infections and pneumonia. For these reasons, one of the more preferred forms, diseases produced by organisms of the genus *Acinetobacter* are selected from a list that consists of bacteremia, meningitis, urinary tract infections, skin and soft tissue infections, surgical site infections and pneumonia

A fifth aspect of the invention refers to an antibody or an active fragment thereof obtainable by immunization of a mammal with the composition of the first or second aspect of the invention, preferably said antibody or active fragment consists of a composition in which preferably said composition is a pharmaceutical composition and said pharmaceutical composition is used as a therapy, particularly for the treatment of infections caused caused by *K. pneumoniae* and/or *P. aeruginosa* and optionally *A. baumannii.*

### METHOD FOR PREPARING AN ACINETOBACTER CELL FOLLOWING THE INVENTION

This specific section constitutes a non-limiting example of a method for preparing an *A. baumannii* strain in accordance to the first aspect of the invention or to any of its preferred embodiments.

First, *A. baumannii* cells are transformed with a vector, preferably a suicide vector, comprising sequences for promoting recombination at the aimed target locus into the *A. baumannii* genome, being such locus any locus of the *A. baumannii* genome comprising sequences suitable to undergo recombination. For example, a suitable locus for integration of an insert by recombination might be: *cysI, trpE, lpxA, lpxC, lpxD, lpxB, lpxK, lpxL, lpxM,* and/or Tn5/Tn7 sites. In a preferred embodiment of this invention, the suitable locus is *cysI.* The *cysI* locus contains the *cysI* ORF, encoding gene, which comprises a 1,644-nt ORF encoding a 547-amino-acid protein that is essential for the biosythesis of the amino-acid cysteine.

The sequences of the vector for promoting recombination are flanking an expression construct comprising at least one or more transcription promoter sequences, one or more ORFs encoding proteins heterologous for *A. baumannii,* and one or more transcription termination sequences. The promoter sequence might be any known sequence in the state of the art able to promote transcription in an *A. baumannii* cell. These promoter sequences are routinely used in bacteriology research. In a prefered embodiment of this invention, the promoter sequence used is a promoter sequence located upstream of the *A. baumannii* ORF encoding the Outer Membrane protein OmpA. The expression construct might comprise several ORFs in tandem, each one flanked by a promoter and a termination, transcriptional termination, sequence upstream and downstream of the ORF, respectively, therefore built for allowing independent expression of each ORF, controlled by a specific promoter. Alternatively, the expression construct might have one or several ORFs within an operon-like structure with polycistronic expression controlled by a common promoter. The ORFs of the expression vector encode proteins or peptides selected by their immunogenic properties, derived from known humans or animal pathogens distinct from *A. baumannii* and whose expression in the *A. baumannii* cell is intended for raising an immune response against pathogens distinct from *A. baumannii* in a human or animal vaccinated with the *A. baumannii* cell. The ORFs of the expression construct might encode for any peptide with immunogenic properties derived from any known pathogen. In a preferred embodiment of this invention, the ORFs of the expression construct encode peptides derived from the Gram-negative bacteria *K. pneumoniae* and/or *P. aeruginosa.* In another prefered embodiment of this invention, the peptides derived from K. pneumoniae comprise sequences derived from the Outer Membrane proteins OmpA and/or OmpK36 from *K. pneumoniae.* In another prefered embodiment of this invention, the peptides derived from P. aeruginosa comprise sequences derived from the outer-membrane proteins OprF and/or OprI from *P. aeruginosa* and/or the protein PcrV of the Type 3 secretion system from *P*. *aeruginosa.* The ORF sequences might include sequences for assisting post-translational processing of the encoded proteins. In particular, the ORFs might contain signal sequences promoting the location of the expressed proeins at the outer-membrane of the *A. baumannii* cells. The nature of these sequences are well-known in the art. In a preferred embodiment of this invention, the signal sequence of the Outer Membrane protein OmpA of *A. baumannii* is used to promote location of the encoded proteins at the outer-membrane of *A. baumannii.* The transcription termination sequences of the expression construct might be any transcription termination sequence able to allow transcription termination in *A. baumannii,* well known in the art.

Second, once the *A. baumannii* cells are transformed with the vector, preferably a suicide vector, recombination between the sequences of the target loci in the host and the sequences in the vector leads to the production of recombinant cells where the sequences of the vector have integrated into the targeted loci on the *A. baumannii* chromosome. This recombination event can be assisted by strategies well-known in the art, like inducing double strand breaks at the insertion site with genome-editing tools, like those based on the CRISPR methodology. In a preferred embodiment of this invention, a first recombination event leads to the insertion of the vector sequences comprising selectable markers and the expression contructs. The selectable markers are used for selection of cells where such first recombination event has ocurred. A second recombination event might lead to recombinant cells where only the expression construct but not the sequences encoding the selectable markers remain integrated at the insertion site. By using as a selection tool the lack of the selectable markes, *A. baumannii* cells where such second recombination event has ocurred can be selected. Once selected, common techniques like PCR and DNA sequencing are used to check whether the integration of the vector sequences into the targeted loci has ocurred as expected.

Third, expression of the proteins encoded by the expression construct in the recombinant *A. baumannii* cells can be analyzed by basic proteomic techniques like Western-Blot or ELISA. Location of the encoded proteins at the Outer Membrane of the recombinant *A*. *baumannii* cells can be analyzed by common proteomic methods previously described in the art such as the methods described in the examples.

Fourth, once the expression of the heterologous proteins analyzed in the recombinant *A. baumannii* cells is considered satisfactory, loss of the LPS is induced in the *A. baumannii* cells by alternative methods like site-directed mutagenesis of a LPS-synthesis gene leading to partial or complete inactivation of LPS-synthesis genes, including the genes selected from the list consisting of *lpxA, lpxB, lpxC, lpxD, lpxK, lpxL* and/or *lpxM*; or by selecting *A*. *baumannii* cells resistant to the antibiotic colistin and screening the cells by common genomic methods like PCR and/or DNA sequencing for mutations leading to partial or complete inactivation of LPS-syntheiss genes, including the genes selected from the list consisting *lpxA, lpxB, lpxC, lpxD, lpxK, lpxL* and/or *lpxM.*

In a particular embodiment of this invention, when the targeted locus for insertion of the recombination events described above is a locus comprising an ORF encoding an LPS-synthesis gene, including genes selected from the list consisting of *lpxA, lpxB, lpxC, lpxD, lpxK, lpxL* and/or *lpxM,* and the recombination leads to partial or complete inactivation of such ORF, the resulting recombinant *A. baumannii* cells are LPS-negative cells due to partial or complete inactivation of LPS-synthesis genes, including the genes selected from the list consisting of *lpxA, lpxB, lpxC, lpxD, lpxK, lpxL* and/or *lpxM.*

It is understood that "infection" in the present invention is that pathology generated by the invasion or colonization of any host tissue by any organisms of the genus *Acinetobacter,* preferably *A. baumannii.*

The term "antigen" in the invention refers to a molecule (generally a protein or polysaccharide) that can induce the formation of antibodies. There many different types of molecules that can act as antigens, such as proteins, peptides, polysaccharides, and more rarely other molecules such as nucleic acids.

The following examples merely illustrate but do not limit the scope of the present invention.

### Examples

### Abbreviatures

*cysI*: gene coding for the sulfite reductase involved in the biosynthesis of the amino-acid L-cysteine.
*trpE*: gene coding for the anthranilate synthase (subunit I) involved in the biosynthesis of the amino-acid tryptophan.
*lpxA*: gene coding for the Acyl-[acyl-carrier-protein]--UDP-N-acetylglucosamine O-acyltransferase involved in the biosynthesis of lipid A, a phosphorylated glycolipid that anchors the lipopolysaccharide to the outer membrane of the cell.
*lpxC*: gene coding for the DP-3-O-acyl-N-acetylglucosamine deacetylase that catalyzes the hydrolysis of UDP-3-O-myristoyl-N-acetylglucosamine to form UDP-3-O-myristoylglucosamine and acetate, the committed step in lipid A biosynthesis.
*lpxD*: gene coding for the DP-3-O-acylglucosamine N-acyltransferase involved in the biosynthesis of lipid A that catalyzes the N-acylation of UDP-3-O-acylglucosamine using 3-hydroxyacyl-ACP as the acyl donor.
*lpxB*: gene coding for the Lipid-A-disaccharide synthase involved in the condensation of UDP-2,3-diacylglucosamine and 2,3-diacylglucosamine-1-phosphate to form lipid A disaccharide, a precursor of lipid A.
*lpxK*: gene coding for the Tetraacyldisaccharide 4'-kinase that transfers the gamma-phosphate of ATP to the 4'-position of a tetraacyldisaccharide 1-phosphate intermediate (termed DS-1-P) to form tetraacyldisaccharide 1,4'-bis-phosphate (lipid IVA). This protein is involved in step 6 of the subpathway that synthesizes lipid IV(A) from (3R)-3-hydroxytetradecanoyl-[acyl-carrier-protein] and UDP-N-acetyl-alpha-D-glucosamine.
*lpxL*: gene coding for the Lipid A biosynthesis lauroyltransferase that catalyzes the transfer of laurate from lauroyl-acyl carrier protein (ACP) to Kdo₂-lipid IV(A) to form Kdo₂-(lauroyl)-lipid IV(A).
*lpxM*: gene coding for the Lipid A biosynthesis myristoyltransferase involved in step 4 of the subpathway that synthesizes KDO(2)-lipid A from CMP-3-deoxy-D-manno-octulosonate and lipid IV(A).
Tn5/Tn7 sites: regions of the *A. baumannii* genome preferred by the transposon Tn5 and Tn7 to be inserted.

### Example 1. Construction and validation of KapaVax candidates, in particular KapaVax2 and KapaVax1 (K1)

KapaVax candidates can be constructed by genome editing of *A. baumannii* using allelic exchange technology for recombinant strain production. Briefly, we selected an *A. baumannii* carrier strain. In particular, we screened for a pan-sensitive clinical isolate of *A*. *baumannii* that can live without LPS as taught in EP2942389A1.The final strain selected in the screening process was named Ab283. The insertion of the expression construct into the *A. baumannii* genome can be assessed by recombination by using different strategies well known in the art such as suicide plasmids or linear DNA fragments. For selection of LPS-negative cells we plate the strains on colistin plates (Ab283 was grown in presence of colistin and selection for colistin-resistant mutant was done) and select for colisitin-resistant mutants. Then, we screen by PCR for large insertions or deletions at the *lpx* genes.

### Preparation of KapaVax2 by insertion of an expression contruct at the cysI locus.

For building of KapaVax2 at the *cysI* locus, after having inserted the constructs at the *cysI* locus in the Ab283 wild-type strain, and having proof of expression and localization of the heterologous antigens at the outer-membrane, we selected an LPS-negative derivative after plating in colistin and selection of an LPS-negative mutant with a ISAba insertion of 1Kb at the *lpxC* ORF.

This candidate KapaVax2 includes both selected *K. pneumoniae* antigens (OmpA and OmpK36) and three *P. aeruginosa* antigens (OprF, OprI and PcrV). Each antigen is expressed from an operon-like construct under the control of the *A. baumannii* OmpA promoter., PcrV was fused to the N-terminus of OprI as a fusion protein.

The expression construct was cloned into a plasmid that encodes one homologous sequence to the target locus *cysI.* This plasmid encodes Kanamycine resistance gene (KanR) as selection marker and an origin of replication unable to replicate in *A. baumannii.* Therefore, once the plasmid is transformed into *A. baumannii* 283 (Ab283) wild-type cells, the plasmid is integrated into the chromosome leading to a KanR phenotype and *cysI* defective phenotype due to the interruption of the ORF by the insertion (7,7Kb). Once the plasmid was inserted into the chromosome, the recombinant Ab283 was grown in presence of colistin in order to select colistin -resistant mutants and therefore obtaining a LPS-negative cell. Then, we screen by PCR for large insertions or deletions at the *lpx* genes.

### Description of KapaVax2 into cysI with pVXD-50 with 2 homology regions

This candidate includes the antigens from *K. pneumoniae* antigens OmpA and ompK36 and the antigens from *P. aeruginosa* OprI, oprF and PcrV under the control of the *A. baumannii* OmpA promoter.

For building Kapavax2 at *cysI* locus, *A. baumannii* cells are transformed with a suicide vector (pVXD-50) comprising sequences for promoting recombination at the *cysI* target locus into the *A. baumannii* genome.

The procedure involves the design of a suicide plasmid that contains the expression construct flanked by homologous regions adjacent of the target gene and selection and counter-selection markers. The ORFs included into the expression construct comprise the sequences derived from the outer-membrane proteins OmpA and OmpK36 from *K. pneumoniae* and the sequences derived from the outer-membrane proteins OprF and OprI from *P. aeruginosa* and the protein PcrV of the Type 3 secretion system from *P. aeruginosa.* The *oprI* and *pcrV* coding sequences are combined in one coding sequence to produce the new chimeric gene *oprI*::*perV*. The four coding sequences are expressed from an operon-like construct under the control of the *A. baumannii* OmpA promoter. The expression construct contains the signal sequence of the outer-membrane protein OmpA from *A. baumannii* upstream of each coding sequence in order to promote location of each antigen at the outer-membrane of *A. baumannii.* The expression construct also contains the transcription termination sequence of the protein OmpA from *A. baumannii.*

The pVXD-50 is constructed by cloning the expression construct flanked by homologous regions adjacent to the Ab283 *cysI* gene (600 bp upstream and 600 bp downstream of the *cysI* gene). The suicide plasmid (non-replicative plasmid in *A. baumannii*) has as selection marker a kanamycin ressitant gene, which induce resistance to kanamycin as well as the gene *sacB* as counter-selection marker.

The pVXD-50 suicide plasmid is introduced into the Ab283 cell by electroporation, matting or chemical transformation and the two-step allelic exchange follows. The first recombination event leads to the insertion of the vector when recombination between the upstream region cloned into the plasmid and the upstream region of the Ab283 genome occurred. Successfully recombinant cells with the plasmid integrated into the genome are selected in kanamycin agar plates. Common techniques like PCR and DNA sequencing are used to check whether the integration of the plasmid into the targeted loci has occurred as expected.

The second recombination event is induced by growing the selected kanamycin-resistant Ab283 cells in presence of sucrose leading to recombinant cells where the vector backbone upstream and downstream of the homologous regions are removed and only the expression construct remains into the recombinant Aab283 cells. The Ab283 cells where such second recombination event has occurred can be obtained by selecting the recombinant Ab283 cells resistant to sucrose and susceptible to kanamycin. Once selected, common techniques like PCR and DNA sequencing are used to check whether the integration of the expression construct into the targeted loci has occurred as expected.

Expression and location at the outer membrane of each antigen has been confirmed by Western Blot and ELISA.

Western blot analysis of whole cell lysates and outer membrane extracts with antigen-specific antibodies has been also used to confirm expression and localization of the heterologous antigens in the *A. baumannii* cell.

For obtaining a LPS-negative cell, the recombinant Ab283 cell expressing the antigens from *K. pneumoniae* and *P. aeruginosa* is grown in presence of colistin to select colistin-resistant mutants.

Then, we screen by PCR for large insertions or deletions at the *lpx* genes. Loss of LPS was confirmed by analysis of the endotoxin levels with the LAL (limulus amebocyte lysate) method.

Preparation of KapaVax1 (K1) by insertion of an expression construct at the IpxA locus.

This candidate includes the selected antigen from *K. pneumoniae* antigens OmpA under the control of the *A. baumannii* OmpA promoter.

For building Kapavax1 at *lpxA* locus, Acinetobacter baumannii cells are transformed with a suicide vector (pVXD-40) comprising sequences for promoting recombination at the *lpxA* target locus into the A. baumannii genome. LpxA protein is essential for the biosynthesis of the LPS in A baumannii.

The procedure involves the design of a suicide plasmid that contains the expression construct flanked by homologous regions adjacent of the target gene and selection and counter-selection markers. The ORF included into the expression construct comprise the sequence derived from the outer-membrane protein OmpA from *K. pneumoniae* controlled by the *A*. *baumannii* OmpA promoter. The signal sequence of the outer-membrane protein OmpA from *A. baumannii* is used to promote location of the OmpA from *K. pneumoniae* at the outer-membrane of *A. baumannii.*

The pVXD-40 is constructed by cloning the expression construct flanked by homologous regions adjacent to the Ab283 *lpxA* gene (2000 bp upstream and 2000 bp downstream of the *lpxA* gene). The suicide plasmid (non-replicative plasmid in *A. baumannii*) has as selection marker a tretracycline resistance cassette , which induce resistance to tetracycline as well as the gene *sacB* as counter-selection marker.

The pVXD-40 suicide plasmid is introduced into the Ab283 cell by electroporation, matting or chemical transformation and the two-step allelic exchange follows. The first recombination event leads to the insertion of the vector when recombination between the upstream region cloned into the plasmid and the upstream region of the Ab283 genome occurred. Successfully recombinant cells with the plasmid integrated into the genome are selected in tetracycline agar plates. Common techniques like PCR and DNA sequencing are used to check whether the integration of the plasmid into the targeted loci has occurred as expected.

The second recombination event is induced by growing the selected tetracycline-resistant Ab283 cells in presence of sucrose leading to recombinant cells where the vector backbone upstream and downstream of the homologous regions are removed and only the expression construct remains into the recombinant Aab283 cells. The Ab283 cells where such second recombination event has occurred can be obtained by selecting the recombinant Ab283 cells resistant to sucrose and susceptible to tetracycline. Once selected, common techniques like PCR and DNA sequencing are used to check whether the integration of the expression construct into the targeted loci has occurred as expected.

The second recombination also leads to complete inactivation of *lpxA* which is essential for the biosynthesis of the LPS in *A baumannii.* Therefore, the second recombination leads to a recombinant LPS-defective Ab283 cell.

Expression of the heterologous protein OmpA from *K. pneumonaie* encoded by the expression construct in the recombinant Ab283 cells is analyzed by basic proteomic techniques like Western-Blot confirming its expression. Location of the *K. pneumoniae* OmpA protein at the outer-membrane of the recombinant *A. baumannii* cells is analyzed by Western-Blot when samples are treated with proteinase K.

### Example 2. Protocol for OMP sensitivity to proteinase K

Objective: to test the location in the of the heterologous antigens from *P*. *aeruginosa* and/or *K. pneumoniae* in the outer membrane of the LPS deficient Acinetobacter baumannii.

Brief description: *A. baumannii* LPS- whole cells, expressing the antigens OmpA and OmpK36 from *K. pneumoniae,* and OprF and OprI::PcrV fusion protein from *P. aeruginosa* are treated with proteinase K. Membrane preparations* from samples treated or not treated (control) are run into 12% acrylamide gels. Consecutively, Western-Blot is carried out using polyclonal antibodies against specific proteins listed above. Proteins located at the membrane surface are degraded upon proteinase K treatment. This part of the protocol has been also performed with lysates of the whole cell and the results show that the polyclonal antibodies recognize cytosolic proteins due to cross-reaction in both samples, treated and not treated with proteinase K. Whereas OMPs are only detected in those samples that have not been treated.

### Methodology

Proteinase K treatment:
   1. Strains: *A. baumannii* LPS- (negative control); KapaVax 2
   2. Preinoculum from a single colony is grown o/n at 30°C with shaking .
   3. Cultures are diluted to OD 0.05 and then grown at 30°C with shaking until OD 2.
   4. 25 ml of culture is concentrated by centrifugation and supernatant is discarded.
   5. Pellet is resuspended in 10 ml of PBS, with proteinase K 250 µg/ml in treated samples.
   6. Incubation at 37°C shaking for 2 hours.
   7. Pellet is concentrated by centrifugation and then resuspended in 10 ml of PBS or PBS+ 3 mM PMFS (protease inhibitor) to stop the reaction in samples treated with proteinase K.
   8. Incubation at room temperature for 10 min.
Membrane preparation
   9. Sonication as follows: 10 seconds at 20% amplitude, 30 seconds pause. This cycle is repeated 7 times.
   10. Ultracentrifugation at 4000 rpm, 8°C for 1 hour.
   11. Discard supernatant and resuspend in 250 µl PBS triton 1%.
   12. Incubate at 4°C o/n for solubilization of membranes.
SDS-PAGE and WB
   13. Sample preparation: 25 µl of membrane preparation + 25 µl loading buffer 4x+ 50 µl of miliQ Water.
   14. Boil at 98°C for 20 minutes.
   15. Load 10 µl on 12% acrylamide gels
   16. Run at 170V for 1 hour.
   17. Wet transference to Nitrocellulose membrane at 360 mA for 1:30 hours
   18. Ponceau staining for loading control.
   19. Blocking with PBS tween 1% milk 5% for 1 hour
   20. O/N incubation at 4°C with primary antibody at 1:5000 in PBS tween 1% milk 5%
   21. Wash membranes with PBS tween 1%. Repeat wash 2 times more.
   22. Incubation with 1:5000 secondary antibody in PBS tween 1% milk 5% at room temperature for 1 hour.
   23. Wash membranes with PBS tween 1%. Repeat wash 5 times more.
   24. Treatment with chemiluminescence reagents for 5 min and visualization.

### EXAMPLE 3. Methods for determination of KapaVax in vivo immunogenicity and potency.

**KapaVax 2** candidates were fermented at laboratory scale and heat inactivated. Immunogenicity has been determined by immunizing C57BL/6 mice (8-10 mice/group) by IM injection with 2 doses on days 0 and 14. KapaVax candidates are adjuvanted with aluminum hydroxide.

The primary variable for evaluation of the immunogenicity of each KapaVax candidate is IgG against each of the antigens measured by indirect ELISA at days 0, 7 and 21 (at baseline, and one week after 1st and 2nd immunizations). As threshold for acceptance of immunogenicity for each candidate is look for an increase in antibody titers against whole-cells of *A. baumannii* (an assay developed and validated for measuring immunogenicity of AcinetoVax candidates) and against each of the heterologous antigens from *K. pneumoniae* and *P. aeruginosa* after 2 immunizations of at least 8-fold compared to baseline titers (pre-immunization titers). All assays include control mice immunized with vehicle alone at the same timepoints.

### In vivo potency in relevant animal models

### KapaVax 2 in vivo proof of concept in murine sepsis model

For in vivo proof-of-concept studies, we employed a murine sepsis model that was previously developed to test *in vivo* potency of our monovalent vaccine against *A*. *baumannii,* AcinetoVax, as described in Garcia-Quintanilla et al. 2014 (PLoS ONE 9(12): e114410).

The KapaVax 2 candidates have been tested in a murine sepsis model against one strain from each species. The following type strains were used, *A. baumannii* ATCC 19606; *P. aeruginosa* PAO1; and *K. pneumoniae* ATCC 43816. Briefly, 6-8 week old C57BL/6 animals (10 animals/group) were immunized. Seven days after the second immunization, the animals were challenged by intraperitoneal injection of an inoculum equivalent to the minimum lethal dose (MLD), previously characterized for each strain in this model. We tested the *in vivo* efficacy in independent challenge experiments against each of the target pathogens.

The animals were monitored for 7 days to determine survival. After exitus or at the end of the follow-up period/ the bacterial load in lungs, spleen and kidneys were determined as a quantitative value of bacterial spread, as well as in the blood to evaluate the bacteremia associated to the experimental sepsis. A separate group of animals (8-10 animals/group) were sacrificed 12 (or 18) hours after infection to evaluate the bacterial load in the different organs and serum levels of TNF-α, IL-Iβ and IL-6 to characterize the inflammatory response developed during infection in vaccinated animals versus unvaccinated control animals.

The primary variables for evaluation of the potency of each candidate were bacterial loads in blood, spleen, kidney, and lungs at 12 (or 18) hours post-infection and *exitus*/7 days post-challenge compared to control mice and survival in the challagne studies (acceptance threshold of 60% for each bacterial species).

### EXAMPLE 4. Protection of AcinetoVax against lethal sepsis caused by diverse A. baumannii clinical isolates

Vaccination with VXD001 (AcinetoVax, a vaccine based on inactivated whole-cells of an LPS-deficient *A. baumannii* strain characterized by the complete inactivation of *lpxC* as taught in EP2942389A1 plus the adjuvant aluminum hydroxide) led to protection against lethal sepsis caused by diverse *A*. *baumannii* clinical isolates. Protection was supported by a rapid humoral response established after the 1st dose, and detected T cell-mediated response (Th1, Th2, Th17) established after the 1st dose and significantly boosted after the 2nd dose.

As shown in figure 1, VXD001 protects against infection with *A. baumannii* clinical isolates. Mice were infected with the indicated strains of *A. baumannii* (ATCC 19606, Ab-154, and Ab-113-16) 7 days after the second immunization with AcinetoVax (day 21) and survival was monitored for 7 days. ATCC 19606 is a urine infection clinical isolate used as reference strain in sepsis and pneumonia models of infection in mice. Ab-154 is a clinical isolate sensitive to carbapenems, from an outbreak at the Hospital Virgen del Rocío of Seville in 2002. Ab-113-16 is a panresistant clinical isolate from a patient who died as a consequence of the 2002 outbreak.

### EXAMPLE 5. Detection of exposure of all heterologous antigens on the OM of A. baumannii 283 LPS- in KapaVax2: successful strategy of chromosomal integration at the cysI locus & expression

Heterologous antigens detected by Western Blot in outer membrane preparations, and showing sensitivity to Proteinase K digestion, supporting exposure on the OM (Fig. 2)

Surface exposure of heterologous antigens is shown in figure 2. Cultures were washed after growth, resuspended in PBS and treated with 0.5 mg/ml of Proteinase K for 1h at 37°C. Western-Blot with polyclonal anti-sera against recombinant protein Pa-OprF in the case of KapaVax 2 (left) and polyclonal anti-sera against recombinant protein Kpn-OmpA in the case of KapaVax 1 (right) . Treatment with Proteinase K is indicated by + (treated) or - (untreated) in each sample). Mb: Outer-Membrane extract; S: extract supernatant. Protocol obtained from Richard N. Besingi and Patricia L. Clark. Nature Protocols. 2015 Dec; 10(12):2074-2080.

In addition, *K. pneumoniae* antigens OmpA and OmpK36 and *P. aeruginosa* antigens OprF and fusion OprI::PcrV have been consistently detected by Western Blot in outer-membrane extracts of KapaVax2@cysI in independent batches Exposure at the OM is supported by their sensitivity to Proteinase K digestion (Fig. 12). The signal detected in Western Blots with polyclonal sera has been consistently higher for the *K. pneumoniae* antigens. In particular, expression of the fusion protein *P. aeruginosa* OprI::PcrV has been the less detectable among all heterologous antigens of KapaVax2@cysI

### EXAMPLE 6. Immunization with KapaVax2 led to significant protection against lethal sepsis produced by K. pneumoniae ATCC43816

Results support that immunity raised by the heterologous antigens was able to fully neutralize infection by a hypervirulent, hypercapsulated strain *K. pneumoniae* ATCC43816.

Figure 3 shows a survival plot of murine sepsis model by *K. pneumoniae* infection. Animals were immunized with 2 doses in alternate weeks with KapaVax2@cysI (N=18), the carrier Ab283 LPS- cells (N=8) or vehicle alone (N=25). IP challenge with the strain ATCC43816 (day 7 after 2nd immunization). The plot merged data from 3 independent experiments, with challenge doses 8.5x10³, 6.7x10³, and 6.7x10³ cfu, respectively. Survival was monitored for 7-12 days. Statistical differences were measured by Log-rank (Mantel-Cox) test (ns stands for non-significant). *K. pneumoniae* ATCC43816 is a hypervirulent, hypercapsulated clinical isolate, producing acute pneumonia and systemic spread

### EXAMPLE 7. KapaVax2 raised specific immunity against the challenge strain of K. pneumoniae and against the K. pneumoniae antigens included in KapaVax2

As shown in figures 4 and 5, KapaVax2 raised specific immunity against the challenge strain of *K. pneumoniae* and against the *K. pneumoniae* antigens included in KapaVax2. Immunogenicity data support contribution of *K. pneumoniae* antigens & requirement for full protection.

Figure 4 shows IgG levels against *K. pneumoniae* cells. ELISA recognition of *K. pneumoniae* ATCC43816 cells by antisera from animals immunized with 2 doses of KapaVax2@cysI (N=10) or vehicle alone (N=10), sampled at day 21. Statistical analysis by 2-tailed Mann-Whitney test (ns, p>0,05; *p<0.05; **p<0.005; ***p<0.001). Red dashed line indicates detection limit.

Figure 5 shows IgG levels against *K. pneumoniae* antigens. ELISA recognition of recombinant proteins KpOmpA and KpOmpK36 by antisera from animals immunized with KapaVax2@cysI (N=4) or vehicle alone (N=4), sampled at day 21 (7 days after 2nd immunization). Statistical analysis by Unpaired 2- tailed T- test (ns p>0,05; *p<0.05; ** p<0.005; ***p<0.001). Red dashed line indicates detection limit.

### EXAMPLE 8. Immunization with KapaVax2 led to significant protection against lethal sepsis produced by P. aeruginosa PA14

Figure 6 shows a survival plot of sepsis model by *P. aeruginosa* infection. Animals were immunized with 2 doses in alternate weeks of the vaccine candidate KapaVax2@cysI, or vehicle alone. IP challenge with the strain PA14 (4.4x10⁶ cfu) was carried out 7 days after second immunization. Survival was monitored for 7 days. N/group= 8. Statistical differences of the survival curves are measured by Log-rank (Mantel-Cox) test. *P. aeruginosa* PA14 is a virulent wound-infection clinical isolate.

### EXAMPLE 9. KapaVax2 raised specific immunity against the challenge strain of P. aeruginosa and against the P. aeruginosa antigens included in KapaVax2

Figure 7 shows IgG levels against *P. aeruginosa* cells. ELISA recognition of *P. aeruginosa* PA14 cells by antisera from animals immunized with KapaVax2@cysI (N=5) or vehicle alone (N=5), sampled at day 21 (7 days after 2nd immunization). Statistical significance: ns p>0,05; *p<0.05; **p<0.005; ***p<0.001. Red dashed line indicates detection limit.

Figure 8 shows IgG levels against *P*. *aeruginosa* antigens. ELISA recognition of recombinant proteins by antisera from animals immunized with 2 doses of KapaVax2@cysI (N=4) or vehicle alone (N=4), sampled at day 21 (7 days after 2nd immunization). Statistical significance: ns p>0,05; *p<0.05; **p<0.005; ***p<0.001. Red dashed line indicates detection limit.

### EXAMPLE 10. Immunization with KapaVax2 led to significant protection against lethal sepsis produced by A. baumannii ATCC19606

Fig. 9. Shows survival plot of sepsis model by *A. baumannii.* Animals (N=8 per group) were immunized with 2 doses in alternate weeks of KapaVax2@cysI or vehicle. IP challenge at day 7 after 2nd immunization, with 4.2 x10⁶ cells of ATCC19606. Survival was monitored for 7 days. *A. baumannii* ATCC19606 is a urine infection clinical isolate used as reference strain in sepsis and pneumonia models of infection in mice.

Fig. 10 shows IgG levels against *A. baumannii* cells. ELISA recognition of *A. baumannii* ATCC 19606 cells by antisera from animals immunized with 2 doses of KapaVax2@cysI (N=9) or vehicle alone (N=9), sampled at day 21 (7 days after 2nd immunization). Boxes show IQR, horizontal line and cross show median and mean, respectively. Error bars extend to the CI95%. Statistical analysis by Unpaired 2-tailed Mann-Whitney test (ns p>0,05; *p<0.05; **p<0.005; ***p<0.001). Red dashed line indicates detection limit.

### EXAMPLE 11. Detection of a panel of global isolates by ELISA supports global strain coverage

KapaVax2 antisera shows specific recognition of 93% of a panel of 15 diverse global clinical isolates *of K. pneumoniae* (73% with higher or similar ELISA titers to that against the challenge strain ATCC43816) KapaVax2 antisera shows specific recognition of 100% of a panel of 15 diverse global clinical isolates of P. aeruginosa (93% with higher or similar ELISA titers to that against the challenge strain PA14)

Fig. 11 shows ELISA IgG titers (1:dilution) of detection of 15 clinical isolates strains of *K. pneumoniae* (9/15 LMICs), *P. aeruginosa* (8/15 LMICs) by antisera from KapaVax2 and Acineto Vax

### EXAMPLE 12. Exposure of A. baumannii immunodominant antigens OmpA and Omp22 at the Outer-Membrane of KapaVax2

Immunodominant OMPs from *A. baumannii* OmpA and Omp22 are detected by Western-Blot from crude extracts made from vaccine batches of KapaVax.

Figure 13 shows Expression of *A. baumannii* antigens in KapaVax2 and AcinetoVax vaccine batches: total lysate preparations from vaccine batches (2x1010 cells/ml). Samples were run on gels with 4- 16% acrylamide gradient. Panels show the signal obtained with monoclonal antibodies raised against OmpA (left) and Omp22 (right).

Figure 14 shows Surface exposure of *A. baumannii* Omp22 at the OM of KapaVax and the carrier cell Ab283 LPS-. Cultures were washed after growth, resuspended in PBS and treated with 0.5 mg/ml of Proteinase K for 1h at 37°C. Western-Blot with a monoclonal antibody raised against Ab-Omp22. Treatment with Proteinase K is indicated by + (treated) or - (untreated) in each sample). Mb: Outer-Membrane extract; S: extract supernatant. Protocol obtained from Richard N. Besingi and Patricia L. Clark. Nature Protocols. 2015 Dec; 10(12):2074-2080.

### References

1. Kurupati P, Teh BK, Kumarasinghe G, Poh CL. Identification of vaccine candidate antigens of an ESBL producing Klebsiella pneumoniae clinical strain by immunoproteome analysis. Proteomics. 2006; 836-44.
2. Kurupati P, Ramachandran NP, Poh CL. Protective Efficacy of DNA Vaccines Encoding Outer Membrane Protein A and OmpK36 of Klebsiella pneumoniae in Mice. Clin. Vaccine Immunol. 2011;18(1):82-8.
3. Hussein KE, Bahey-el-din M, Sheweita SA. Immunization with the outer membrane proteins OmpK17 and OmpK36 elicits protection against Klebsiella pneumoniae in the murine infection model. Microbial Pathogenesis 2018; 119:12-8.
4. Babu L, Uppalapati SR, Sripathy MH, Reddy PN. Evaluation of Recombinant Multi-Epitope Outer Membrane Protein-Based Klebsiella pneumoniae Subunit Vaccine in Mouse Model. Front. Microbiol. 2017; 8:1-12.
5. Gartner T, Baeten M, Otieno S, Revets H, Baetselier P et al. Mucosal prime-boost vaccination for tuberculosis based on TLR triggering OprI lipoprotein from Pseudomonas aeruginosa fused to mycolyl-transferase Ag85A. Immunology letters. 2007; 111:26-35.
6. Cote-Sierra J, Bredan A, Toldos CM, et al. Bacterial lipoprotein- based vaccines induce tumor necrosis factor-dependent type 1 protective immunity against Leishmania major. Infection and Immunity 2002;70(1):240-8.
7. Rau H, Revets H, Cornelis P, Titzmann A, Ruggli N et al. Efficacy and functionality of lipoprotein OprI from Pseudomonas aeruginosa as adjuvant for a subunit vaccine against classical swine fever. Vaccine. 2006;24:4757-4768.
8. Finke M, Duchene M, Eckhardt A, Domdey H. Protection against Experimental Pseudomonas aeruginosa Infection by Recombinant P. aeruginosa Lipoprotein I Expressed in Escherichia coli. Infection and Immunity. 1990;58(7):2241-4.
9. Finke M, Muth G, Reichhelm T, Thoma M, Duchene M et al. Protection of Immunosuppressed Mice against Infection with Pseudomonas aeruginosa by Recombinant P. aeruginosa Lipoprotein I and Lipoprotein I-Specific Monoclonal Antibodies. Infection and Immunity. 1991; 59:1251-1254.
10. von Specht BU, Licking HC, Schmitt A, Hungerert KD, Domdey H. Safety and immunogenicity of a Pseudomonas aeruginosa outer membrane protein I vaccine in human volunteers. Vaccine. 1996;14(12):1111-7.
11. Moriyama K, Wiener-Kronish JP, Sawa T. Protective effects of affinity-purified antibody and truncated vaccines against Pseudomonas aeruginosa V-antigen in neutropenic mice. Microbiol. Immunol. 2009; 53: 587-594.
12. Sawa T, Yahr TL, Ohara M, Kurahashi K, Gropper MA, Wiener-Kronish JP, Frank DW. Active and passive immunization with the Pseudomonas V antigen protects against type III intoxication and lung injury. Nat. Med. 1999; 5:392-398.
13. Milla CE, Chmiel JF, Accurso FJ, VanDevanter DR, Konstan MW, Yarranton G, et al. Anti-PcrV antibody in cystic fibrosis: a novel approach targeting Pseudomonas aeruginosa airway infection. Pediatric Pulmonology 2014; 49:650-8.
14. Sawa T, Ito E, Nguyen VH, Haight M. Anti-PcrV antibody strategies against virulent Pseudomonas aeruginosa. Hum. Vaccin. Immunother. 2014; 10:2843-52.
15. Aguilera-Herce J, Garcia-Quintanilla M, Romero-Flores R, McConnell MJ, Ramos-Morales F. A live Salmonella vaccine delivering PcrV through the type III secretion system protects against Pseudomonas aeruginosa. mSphere 2019; 4:e00116-19.
16. Meynet E, Laurin D, Luc J, Camara B, Toussaint B, Le A. Killed but metabolically active Pseudomonas aeruginosa-based vaccine induces protective humoral- and cell-mediated immunity against Pseudomonas aeruginosa pulmonary infections. Vaccine. 2018; 36(14): 1893-1900.
17. Hamaoka S, Naito Y, Katoh H, Shimizu M, Kinoshita M et al. Efficacy comparison of adjuvants in PcrV vaccine against Pseudomonas aeruginosa pneumonia. Microbiology and Immunology. 2017; 61:64-67.
18. Francois, B, Luyt CE, Dugard A, Wolff M. et al. Safety and pharmacokinetics of an anti-PcrV PEGylated monoclonal antibody fragment in mechanically ventilated patients colonized with Pseudomonas aeruginosa: a randomized, double-blind, placebo-controlled trial. Critical Care Medicine. 2012; 40, 2320-2326.
19. Ali, S, Yu XQ, Robbie GJ, Shoemaker K. et al. Phase 1 study of MEDI3902, an investigational anti- Pseudomonas aeruginosa PcrV and Psl bispecific human monoclonal antibody, in healthy adults. Clinical Microbiology and Infection. 2018. http://dx.doi.org/10.1016/j.cmi.2018.08.004 pii: S1198-743X(18)30576-7.
20. Guilleland Jr,Parker MG, Matthews JM et al. Use of a Purified Outer Membrane Protein F (Porin) Preparation of Pseudomonas aeruginosa as a Protective Vaccine in Mice. Infection and Immunity. 1984;44:49-54.
21. Hassan R, El-naggar W, El-aziz AM, Saaban M, Kenawy HI, Ali YM. Immunization with outer membrane proteins (OprF and OprI) and flagellin B protects mice from pulmonary infection with mucoid and nonmucoid Pseudomonas aeruginosa. J. Microbiol. Immunol. Infect. 2018; 51(3):312-320.

## Claims

1. A composition comprising:
a. an *A. baumannii* strain deficient in lipopolysaccharide (LPS) **characterized by** the partial or complete inactivation of one or various cellular nucleic acid molecules that encode endogenous LPS biosynthesis genes; wherein the *A. baumannii* strain deficient in lipopolysaccharide (LPS) is **characterized by** the partial or complete inactivation of the genes selected from the list consisting of *lpxA, lpxB, lpxC, lpxD, lpxK, lpxL* and/or *lpxM*; and wherein the *A. baumannii* strain deficient in lipopolysaccharide (LPS) expresses one or more heterologous antigens with targeted location at the Outer Membrane; and/or
b. an outer membrane vesicle (OMV) derived from an *A. baumannii* strain deficient in lipopolysaccharide (LPS) as defined in paragraph a) above.

2. The composition according to claim 1, wherein the strain is modified to express the one or more heterologous antigens with targeted location at at the Outer Membrane by insertion of an expression construct coding for the one or more heterologous antigens at a locus in the *A. baumannii* chromosome that can incorporate an insert by recombination, selected from the list consisting of: *cysI, trpE, lpxA, lpxC, lpxD, lpxB, lpxK, lpxL, lpxM,* and/or the Tn5/Tn7 sites.

3. The composition according to claim 1 or 2, wherein the ORFs of the one or multiple copies of antigenic outer-membrane heterologous proteins comprise signal sequences of the outer-membrane protein OmpA of *A. baumannii* to locate the encoded heterologous antigens at the outer-membrane of *A. baumannii.*

4. The composition according to any of claims 1 to 3, wherein the heterologous antigens expressed at the Outer Membrane of the *A. baumannii* strain deficient in lipopolysaccharide (LPS) are at least derived from *K. pneumoniae* and/or *P. aeruginosa.*

5. The composition according to claim 4, wherein the heterologous antigens expressed at the Outer Membrane of the *A. baumannii* strain deficient in lipopolysaccharide (LPS) are at least derived from *K. pneumoniae* and are selected from the list consisting of Kp-OmpA and Kp-Ompk36.

6. The composition according to claim 4, wherein the heterologous antigens expressed at the Outer Membrane of the *A. baumannii* strain deficient in lipopolysaccharide (LPS) are at least derived from *P. aeruginosa* and are selected from the list consisting of Pa-OprF, Pa-OprI, and Pa-OprI:PcrV.

7. The composition according to claim 4, wherein the heterologous antigens expressed at the Outer Membrane of the *A. baumannii* strain deficient in lipopolysaccharide (LPS) are at least derived from *K. pneumoniae* and are selected from the list consisting of Kp-OmpA and Kp-Ompk36, and are derived from *P. aeruginosa* and are selected from the list consisting of Pa-OprF, Pa-OprI and Pa-OprI:PcrV.

8. The composition according to claim 7, wherein the *A. baumannii* strain deficient in lipopolysaccharide (LPS) further comprises the expression of *A. baumannii* antigens Ab-OmpA and Ab-Omp22.

9. The composition according to any of claims 1 to 8, wherein the *A. baumannii* strain deficient in lipopolysaccharide (LPS) is **characterized by** the partial or complete inactivation of the genes selected from the list consisting of *lpxA, lpxB, lpxC.*

10. The composition according to any of claims 1 to 9, for use in therapy.

11. The composition according to any of claims 1 to 9, for use in delivering bacterial Outer Membrane antigens in a subject, preferably a human subject, in need thereof.

12. The composition according to any of claims 1 to 9, for use in delivering bacterial Outer Membrane antigens for inducing an immunological protective response at least against *K. pneumoniae* and/or *P. aeruginosa* and optionally *A. baumannii* in a subject, preferably a human subject, in need thereof.

13. A vaccine composition comprising an *A. baumannii* strain deficient in lipopolysaccharide (LPS) as defined in any of claims 1 to 9.

14. The vaccine according to claim 13, wherein the vaccine comprises from about 10⁶ to about 10¹² *A. baumannii* strains deficient in lipopolysaccharide (LPS), preferably about 10⁹ *A. baumannii* strains deficient in lipopolysaccharide (LPS).

15. The vaccine according to any of claims 13 or 14, wherein the vaccine comprises an adjuvant, preferably Al(OH)₃.
